# NEW EUROPEAN PATENT SPECIFICATION

(11) **EP 0 140 308 B2**
(45) Date of publication and mention of the opposition decision: **17.10.2001**
(45) Mention of the grant of the patent: 27.01.1993
(21) Application number: 84112647.7
(22) Date of filing: 19.10.1984
(51) Int. Cl.: C12N 15/11, C12N 1/20

(54) **Regulation of gene expression by employing translational inhibition utilizing mRNA interfering complementary RNA**
Regulierung von Gen-Expression durch Translationshemmung unter Verwendung von m-RNS hinderndem Komplementär-RNS
Réglage d'expression de gène par inhibition de la traduction en utilisant de l'ANR complémentaire interferrant avec le m-ANR

(30) Priority: 20.10.1983 US 543528; 01.03.1984 US 585282
(43) Date of publication of application: 08.05.1985
(60) Divisional application: 91111983.2
(73) Proprietor: THE RESEARCH FOUNDATION OF STATE UNIVERSITY OF NEW YORK, Albany, New York 12201-0009 (US)
(72) Inventor: Inouye, Masayori, Setauket, NY 11733 (US); Mizuno, Takeshi, Machida-shi Tokyo 194 (JP); Chou, Mei-Yin, Coram, NY 11727 (US)
(74) Representative: VOSSIUS & PARTNER

(56) References cited:
- WO-A-83/01451
- CELL, vol. 34, September 1983, pages 683-691, MIT; R.W: SIMONS et al.: "Translational control of IS10 transposition"
- PROCEEDINGS OF THE JAPAN ACADEMY, series B, vol. 59, December 1983, pages 335-338; T. MIZUNO et al.: "Regulation of gene expression by a small RNA transcript (micRNA) in Escherichia coli K.12"
- The EMBO Journal, vol.2, no.1, 1983, pp 93-98
- Cell, vol.34, 1983, pp 683-91
- Nature, vol. 294, 1981, pp 623-26
- Methods in Enzymology, vol.68, 1979, pp 482-93
- Cell, vol. 42, 1985, pp 129-38
- Gene, vol.72,1988, pp 25-34
- Deposition upon oral examination of Dr. Masayori Inouye, 16.09.93., extracts from volumes I and II
- Izant and Weintraub, Cell, 1984,36 : 1007-1015

## Description

### BACKGROUND OF THE INVENTION

The control or regulation of the gene expression of the genetic material of cellular material or an organism has received special attention by scientists and in special circumstances, employing recombinant DNA and other techniques, has been achieved. For example, in the PCT Patent Application WO 83/01451 published April 23, 1983, there is disclosed a technique employing an oligonucleotide, preferably in phosphotriester form, having a base sequence substantially complementary to a portion of messenger ribonucleic acid mRNA coding for a biological component of an organism. This oligonucleotide is introduced into the organism and, due to the complementary nature of the oligonucleotide and the messenger ribonucleotide, the two components hybridize under appropriate conditions to control or inhibit synthesis of the organism's biological component coded for by the messenger ribonucleotide. If the biological component is vital to the organism's viability, then the oligonucletoide could act as an antibiotic. A related technique for the regulation of gene expression in an organism is described in an article appearing in Cell, Vol. 34, p: 683 of September, 1983. The disclosures of the above-identified publications are herein incorporated and made part of this disclosure.

As indicated hereinabove, it is known that the expression of certain genes is capable of being regulated at the level of transcription. Transcriptional regulation is carried out either negatively (repressors) or positively (activators) by a protein factor. It is also known that certain specific protein factors regulate translation of specific mRNAs. Also, as indicated hereinabove, it has become evident that RNAs are involved in regulating the expression of specific genes and it has been reported that a small RNA transcript of 174 bases is produced, upon growing Escherichia coli in a medium of high osmolarity, which inhibits the expression of the gene for an outer membrane protein (OmpF protein), see "Regulation of Gene Expression by a Small RNA Transcription (micRNA) in E. coli K12" , Proc Jap. Acad., 59, 335-338 (1983). The inhibition of OmpF protein production by the small RNA transcript (micRNA, i.e. mRNA interfering complementary RNA) is likely due to the formation of the hybrid between the micRNA and the ompF mRNA over a region of approximately 80 bases including the Shine-Dalgarno sequence and the initiation codon. A similar regulation by a small complementary RNA has also been described for the Tn10 transposase, see Simons et al "Translational Control of IS10 Transposition", Cell, 34, 683-691 (1983). In this case, however, the gene for the transposase and the gene for the micRNA are transcribed in opposite directions off the same segment of DNA such that the 5'-ends of the transcripts can form a complementary hybrid. The hybrid is thought to inhibit translation of the transposase mRNA. However, the transposase situation is in contrast to the ompF situation in which the ompF gene and the micRNA gene (micF) are completely unlinked and map at 21 and 47 minutes, respectively, on the E. coli chromosomes.

It is an object of this invention to provide a technique useful for the regulation of gene expression of the genetic material making up an organism.

It is another object of this invention to provide transformed organisms having special properties with respect to the gene expression of the genetic material making up said organisms.

It is yet another object of this invention to provide DNA or other genetic material, such as plasmids containing the DNA, which transcribes to an RNA which is complementary to and capable of binding or hybridizing to the mRNA of the genetic material into which said DNA or plasmid containing the DNA is introduced.

How these and other objects of this invention are achieved will become apparent in the light of the accompanying disclosure and with reference to the accompanying drawings wherein:
Fig. 1 describes the construction of a subclone of a gene and various plasmids carrying the promoter region therefor;
Fig. 2 sets forth the nucleotide sequence of the promoter region and upstream of a gene, specifically the ompC gene;
Fig. 3 illustrates the hybrid formation between certain RNA in accordance with the practices of this invention;
Fig. 4 illustrates the homologous sequences between certain genes, specifically micF and the ompC genes; and
Fig. 5 illustrates a possible model for the role of RNA, specifically micF RNA useful in and in accordance with the practices of this invention.
Fig. 6 illustrates the construction of mic vector pJDC402 and mic(Ipp).
Fig. 7 illustrates the homology between the ompC mRNA and the Ipp mRNA
Fig. 8 illustrates fragments used to construct mic(ompA) genes.

### SUMMARY OF THE INVENTION

Gene expression of the genetic material of cellular material or an organism in accordance with the practices of this invention is regulated, inhibited and/or controlled by incorporating in or along with the genetic material of the cellular material or organism DNA or other genetic material which transcribes to an RNA which is complementary to and capable of binding or hybridizing to the mRNA of the genetic material of said organism or cellular material. Upon binding to or hybridization with the mRNA, the translation of the mRNA is prevented with the result that the product, such as protein material coded for by the mRNA is not produced. In the instance where the mRNA translated product, e.g. protein, is vital to the growth of the organism or cellular material, the organism or cellular material so transformed or altered becomes, at least, disabled.

In accordance with the practices of this invention there has been constructed a mic system designed to regulate the expression of a gene. More particularly, one can construct in accordance with the practices of this invention an artificial mic system to regulate the expression of any specific gene in E. coli.

Further, in accordance with the practices of this invention, a micRNA system for a gene is constructed by inserting a small DNA fragment from the gene, in the opposite orientation, after a promoter. Such a system provides a way, heretofore unknown, for specifically regulating the expression of any gene. More particularly, by inserting the micDNA fragments under the control of an inducible promoter, particularly as embodied in E. coli, the expression of essential E. coli genes can be regulated. It would appear, therefore, that in accordance with the practices of this invention, the inducible lethality thus-created may be an effective tool in the study of essential genes.

Hereinafter, in accordance with the practices of this invention, there is described the construction of an artificial mic system and the demonstration of its function utilizing several E. coli genes. The mic system in accordance with this invention is an effective way to regulate the expression of specific prokaryotic genes. This invention accordingly provides the basis for accomplishing similar regulation of biologically important genes in eukaryotes. For example, the mic system can be used to block the expression of harmful genes, such as oncogenes and viral genes, and influence the expression of substantially any other gene, harmful or otherwise.

The practices of this invention are applicable to both procaryotic and eucaryotic cellular materials or micro-organisms, including bacteria and viruses, and are generally applicable to organisms, which contain genetic material which are expressed.

Accordingly, in the practices of this invention from a genetic point of view as evidenced by gene expression, new organisms are readily produced. Further, the practices of this invention provide a powerful tool or technique for altering gene expression of the genetic material making up organisms and the like so as to make such organisms disabled or incapable of functioning normally or imparting special properties thereto. The DNA material employed in the practices of this invention can be incorporated into the organisms to be treated or effected, such as by direct introduction into the nucleus of a eucaryotic organism or by way of a plasmid or suitable vector containing the special DNA of this invention in case of a procaryotic organism.

### DETAILED DESCRIPTION OF THE INVENTION

By way of further background of the practices of this invention, it has been found that the expression of the genes for the major outer membrane proteins, OmpF and OmpC, of Escherichia coli are osmoregulated. The ompC locus was found to be transcribed bidirectionally under conditions of high osmolarity, and the upstream transcript RNA of approximately 170 bases was found to inhibit the production of OmpF protein. This RNA (micRNA) has a long sequence which is complementary to the 5'-end region of the ompF mRNA that includes the ribosome-binding site and the coding region of the first nine amino acid residues of pro-OmpF protein. Thus, it is proposed that micRNA inhibits the translation of ompF mRNA by hybridizing with it. This novel mechanism can account for the observation that the total amount of the OmpF and of the OmpC proteins is always constant in E. coli.

The major outer membrane proteins of Escherichia coli, OmpF and OmpC, are essential proteins which function as passive diffusion pores for small, hydrophilic molecules. These matrix porin proteins are encoded by the structural genes ompF and ompC, which map at 21 and 47 min on the E. coli chromosome, respectively, see Reeves, P. in Bactrial Outer Membranes: Biogenesis and Function (ed. Inouye, M.) 255-291 (John Wiley and Sons, New York, 1979). The expression of these genes is regulated by the osmolarity of the culture medium. There is a strict compensatory production of both proteins: as the osmolarity of the culture medium increases, the production of OmpF protein decreases, while the production of OmpC protein increases so that the total amount of the OmpF plus OmpC proteins is constant. This osmoregulation of the ompF and ompC genes is controlled by another unlinked locus, ompB, which maps at 74 min, see Hall, M.N. & Silhavy, T.J., J. Mol. Biol. 146, 23-43 (1981) and Hall, M.N. & Silhavy, T.J., J.Mol. Biol. 151, 1-15 (1981). The ompB locus contains two genes called ompR and envZ. The DNA sequences of both genes have been determined and their gene products have been characterized, see Wurtzel, E.T. et al., J. Biol. Chem. 257, 13685-1391 (1982) and Mizuno, T., et al., J. Biol. Chem. 257, 13692-13698 (1982). The EnvZ protein is assumed to be a membrane receptor protein which serves as an osmosensor and transmits the signal from the culture medium to the OmpR protein. The OmpR protein then serves as a positive regulator for the expression of the ompF and ompC genes. The ompF and ompC genes were sequenced, and extensive homology was found in their coding regions, while there was very little homology in their promoter regions. It was during the course of the characterization of the ompC gene, that the novel regulatory mechanism of gene expression mediated by a new species of RNA called mRNA interfering complementary RNA (micRNA) in accordance with this invention was discovered and/or elicited. MicRNA is produced from an independent transcriptional unit (the micF gene). This gene is located immediately upstream of the ompC gene but is transcribed in the opposite direction. The 174-base micRNA blocks the translation of the ompF mRNA by hybridizing to it. Since the production of micRNA is assumed to be proportional to the production of ompC mRNA, this regulatory mechanism appears to be a very efficient way to maintain a constant total amount of OmpF and OmpC proteins.

### A DNA Fragment Suppressing ompF Expression

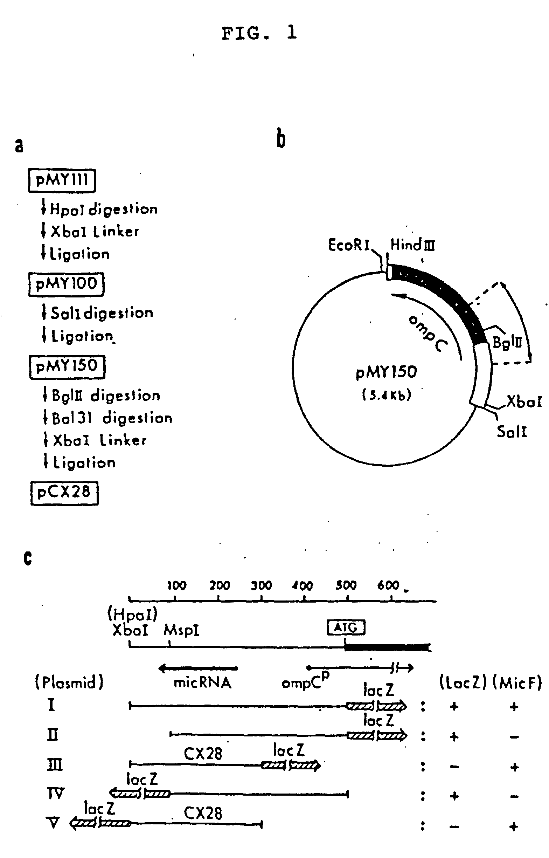

While characterizing the ompC promoter, it was found that a DNA fragment of approximately 300 bp, located upstream of the ompC promoter, completely blocked the production of OmpF protein when OmpF⁺ cells were transformed with a multi-copy plasmid harboring this DNA fragment. For this experiment, plasmid pMY150 was constructed from the original ompC clone, pMY111, see Mizuno, T. et al, J.Biol. Chem. 258, 6932-6940 (1982), by changing the Hpal sites of pMY111 to Hbal sites followed by removal of the 1.1 kb Sall fragment as described in Fig. 1a of Fig. 1.

In Fig. 1 there is shown the construction of the subclone of the ompC gene and various plasmids carrying the ompC promoter region.
(a) Schematic presentation of the subcloning of the ompC gene. Plasmid pMY111 carrying a 2.7 Kb E. coli chromosomal DNA in pBR322 was described previously. The plasmid (1 ug of DNA) was digested with Hpal and religated in the presence of an Xbal linker (CTCTAGAG, 150 p mole). Thus, ca. 400 bp Hapl fragment was removed and a unique Xbal site was newly created (pMY100). Plasmid pMY100 (1 ug of DNA) was further digested with Sall and religated to remove a 1.1 kb Sall fragment (pMY150). In order to obtain an ompC promoter fragment of different sizes, plasmid pMY150 was digested by Bal 31 nuclease after cleavage of the unique BgIII site (see Fig 1b), subsequently the plasmid was religated in the presence of an Xbal linker. Plasmid pCX28 thus constructed is one of clones carrying approximately 300-bp Xbal-Xbal fragment as shown in Fig. 1b.
(b) Simplified restriction map of the plasmid pMY150 carrying the entire ompc gene. The 1.8 Kb HindIII-Sall fragment (boxed region) in pBR322 contains the entire ompC coding region as well as the 5'- and 3'-non-coding region. Transcription of the ompC gene proceeds in the direction shown by an arrow. A bidirectional arrow indicates an approximate deleted region (ca. 600 bp) for plasmid pCX28.
(c) Various *β*-galactosidase (lacZ) gene fusions to the DNA fragments derived from the ompC promoter and its upstream region: Plasmid I, 507-bp Xbal-Rsal fragment was isolated from pMY150 (an Rsal site is present just downstream of the ATG codon), and inserted between Xbal-Smal sites of plasmid pICIII which is derived from plasmid pINIII carrying the lacz gene. During the litigation, a HindIII linker was inserted between the Rsal and Smal ligation site. The Xbal-HindIII fragment was isolated from the plasmid thus constructed and reinserted into plasmid pKM005 to create a lacZ gene fusion in the right reading frame. Characteristic features of plasmids plCIII and pKM005 were described previously. Plasmids II and IV carrying approximately 430-bp Mspl-BamHI fragment was isolated from clone I (a BamHI site is present just downstream of the ATG codon for the *β*-galactosidase coding sequence in plasmid I), and treated with S1 nuclease to create blunt ends. After adding Xbal linkers at both ends, the Xbal-XbaI fragment thus obtained was inserted into plasmid pKM005 at its Xbal site in the possible two orientations. Plasmids III and V, an approximately 300 bp XbaI-XbaI fragment was isolated from plasmid pCX28 (Fig. 1a) and inserted into plasmid pKM005 at its Xbal site in the two possible orientations. These plasmids (I-V) were transformed into a lacZ deletion strain SB4288 (F⁻ recA thi-I relA mal24 spcI2 supE-50 proB lac), and those *β*-galactosidase activities were tested on MacConkey plates (Difco). Results are shown as LacZ⁺ or LacZ⁻. Ability of these clones to inhibit the expression of OmpF protein are also shown as MicF⁺ or MicF⁻.

The plasmid, pMY150 (Fig. 1b) contains the entire coding region of the ompC gene and approximately 500 bp of upstream sequences including the ompC promoter and the DNA encoding the 5'-end untranslated region of ompC mRNA. In order to obtain an ompC promoter fragment of different sizes, pMY150 was digested by Bal31 nuclease at the unique Bglll site, followed by the addition of Xbal linkers. The plasmids constructed in this manner carry Xbal fragments that vary in size due to the position of the Xbal site furthest from the Sall site (see Fig. 1b). The different Xbal fragements were subsequently transferred to a promoter-cloning vector, pKM005 which can express the lacZ gene only when a promoter fragment is inserted in the right orientation into its unique Xbal site. These experiments revealed that transcription of the ompC gene initiates at a site located between 390 and 440 bp downstream from the upstream Xbal site (originally Hpal site). Surprisingly, E. coli transformed with these pKM005 derivatives, including the clone of the shortest Xbal fragment of only 300 bp, CX28 (subcloned from pCX28; Fig. 1a and b), lost the ability to produce OmpF protein. OmpF protein was clearly produced in the host cells (ompB⁺ ompF⁺ ompC⁺), while the same cells carrying the clone of the CX28 fragment were not able to produce OmpF protein. The same effect could be observed with cells harboring a clone of a longer fragment such as plasmid I in Fig. 1c. In this clone the lacZ gene was fused immediately after the initiation codon of the ompC gene resulting in the LacZ⁺ phenotype of the cells carrying this plasmid. However, when the Xbal-MspI fragment of 87 bp was removed from plasmid I, the cells carrying the resulting plasmid (plasmid II in Fig. 1c) were able to produce OmpF protein. It should be mentioned that a similar DNA fragment of 430 bp in length containing the upstream region of the ompF gene did not block the production of both OmpF and OmpC proteins.

### DNA sequence Homology Between CX28 and the ompF Gene

The results described above demonstrate that the stretch of DNA approximately 300 bp long, located upstream of the ompC promoter, is able to block ompF expression. In order to elucidate the function of this DNA fragment (CX28), the DNA sequence of this region was determined.

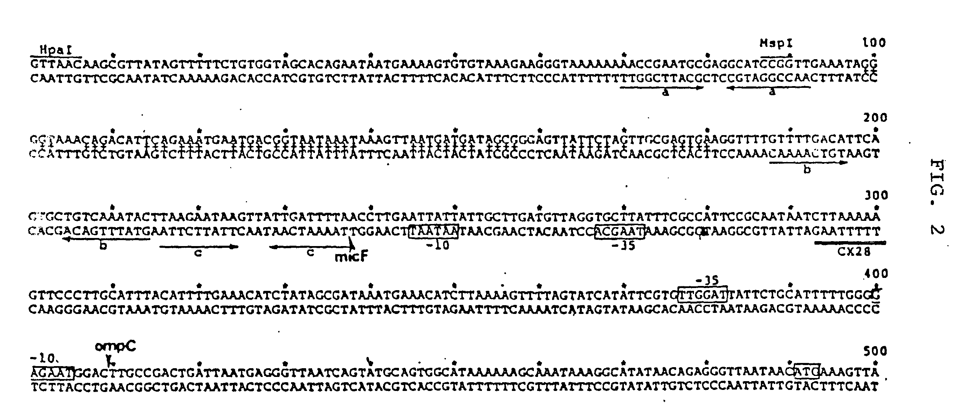

Reference is now made to Fig. 2 which shows the nucleotide sequence of the promoter region and upstream of the ompC gene. Restriction DNA fragments prepared from pMY111 or pMY150 were labeled at their 3'-end by the method of Sakano et al., Nature, 280, 288-294 (1979), using [α-³²P] dNTP's and DNA polymerase I large fragment (Klenow fragment). Singly end-labeled DNA fragment was obtained by digestion with a second restriction enzyme. DNA sequence were determined by the method of Maxam and Gilbert, Methods in Enzymology 65, 499-560 (1981), using 20%, 10% and 6% polyacrylamide gels in 7 M urea. The RNA polymerase recognition site (-35 region) and the Pribnow box (-10 region) for the ompC and micF promoter, as well as the initiation codon of the ompC gene are boxed in figure 2. The transcriptional initiation sites were determined by S1 nuclease mapping for the ompC and micF genes.

Fig. 2 shows the DNA sequence of 500 bp from the Xbal site (originally Hpal) to the initiation codon, ATG, of the ompC gene. The DNA sequence downstream of residue 88 was determined previously. It was found that the sequence from residue 99 to 180 (Fig. 2) has 70% homology with the 5'-end region of the ompF mRNA which includes the Shine-Dalgarno sequence, the initiation codon, and the codons for the first nine amino acid residues of pro-OmpF protein (bases marked by + are homologous to the ompF sequence). A plausible model to explain the above result is that the 300-bp CX28 fragment (Fig. 1c) contains a transcription unit which is directed towards the region upstream of the ompC gene so that the RNA transcript from this region has a sequence complementary to the ompF mRNA. The hybridization between the two RNAs thus blocks the production of OmpF protein.

### Existence of a New Transcription Unit

To determine whether the CX 28 fragment contained an independent transcription unit oriented in a direction opposite from the ompC gene, the lacZ gene was fused at two different sites within the CX28 fragment. In plasmid V, the CX28 fragment was inserted in the opposite orientation with respect to plasmid III (Fig. 1c). This clone was still fully active in suppressing the production of OmpF protein, although it did not produce *β*-galactosidase (LacZ⁻) (see Fig. 1c). When the fusion junction was shifted to the Mspl site at nucleotide 88 (Fig. 2, also see Fig. 1c), the newly constructed clone (plasmid IV) was capable of producing *β*-galactosidase. However, this plasmid was no longer able to suppress the production of OmpF protein. Although this plasmid contains additional DNA (approximately 200 bp) at the upstream of the lacZ and the CX28 sequences (from residue 300 to 500; Fig. 2), this should not affect the functions of the CX28 fragment since plasmid V is fully active in the suppression of OmpF protein production. These results demonstrate that there is a transcription unit in the CX28 fragment which is independent from the ompC gene promoter and that the CX28 fragment and the ompC gene are transcribed in divergent directions. The fact that plasmid IV can produce *β*-galactosidase and plasmid V does not, indicates that the CX28 transcription unit terminates between residue 1 and 88 (Fig. 1c). In fact, a very stable stem-and-loop structure can form between nucleotides 70 and 92 (arrows with letter a in Fig. 2) which is followed by oligo-[T]. This structure is characeristic of p-factor independent transcription termination sites in prokaryotes. The ΔG value for this structure was calculated to be -12.5 Kcal according to Salser, W., Cold Spring Harbor Symp. Quant. Biol. 13, 985-1002 (1977).

The initiation site for the CX28 transcript was positioned at nucleotide 237 (Fig. 2) by S1-nuclease mapping. This result indicates that the CX28 DNA fragment is transcribed to produce a transcript of 174 nucleotides. This was further proven by Northern blot hybridization. In the RNA preparation extracted from cells carrying plasmid III (Fig. 1c), an RNA species is clearly observed to hybridize with the CX28 fragment, which migrates a little slower than 5S RNA. In the control cells, only a small amount of the same RNA was detected. The size of the RNA (CX28 RNA) was estimated on gel to be approximately 6S which is in very good agreement with the size estimated from the sequence (174 bases).

### Function of the CX28 RNA

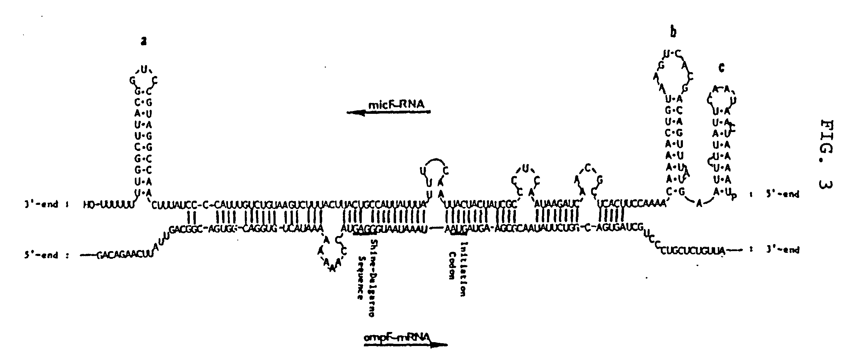

As pointed out earlier, the CX28 DNA fragment has extensive homologies with a portion of the ompF gene. Thus, part of CX28 RNA is complementary to the ompF mRNA and can form an extremely stable hybrid with the ompF mRNA as shown in Fig. 3. The ΔG value for this hybrid formation was calculated to be -55.5 Kcal. Forty-four bases of the 5'-end untranslated region of ompF mRNA, including the Shine-Delgarno sequence for ribosome-binding and 28 bases from the coding region, are involved in the hybrid formation. This hybrid structure is sandwiched by the two stable stem-and-loop structures of the CX28 RNA; one for the 3'-end p-independent transcription termination signal (loop a) and the other at the 5'-end (loop b). The ΔG values for loops a and b were calculated to be -12.5 and -4.5 Kcal, respectively.

Referring now to Fig. 3 of the drawings, there is illustrated therein hybrid formation between micF and ompf mRNA. The sequence of micF RNA corresponds to the sequence from residue 237 to 64 in Fig. 2. The ompF mRNA sequence was cited from Inokuchi, K. et al., Nucleic Acids Res. 10, 6957-6968 (1982). The ΔG values for the secondary structures a, b and c were calculated to be -12.5, -4.5 and +2.9 Kcal, respectively.

In Fig. 3 another loop (loop c) is shown. This loop however, is unlikely to be formed because of its ΔG value (+2.9 Kcal). It appears that the formation of the hybrid blocks the translation of ompF mRNA. This would explain why clones carrying the CX28 DNA fragment suppress the production of OmpF protein. Thus, CX28 RNA is designated as the mRNA-interferring complementary RNA for ompF (micRNA for ompF) and the gene is designated micF. It should be noted that when loop a was eliminated by fusing the micF gene with the lacZ gene, the MicF function was abolished (plasmid IV, Fig. 1c). This may be due to the stability of the micF RNA or alternatively due to the requirement of loop a for the MicF function.

It seemed of interest to examine whether the micF gene is under the control of the ompB locus as is the ompC gene. Various lacZ clones were therefore put into four different ompB mutants. Reference is now made to Table I.

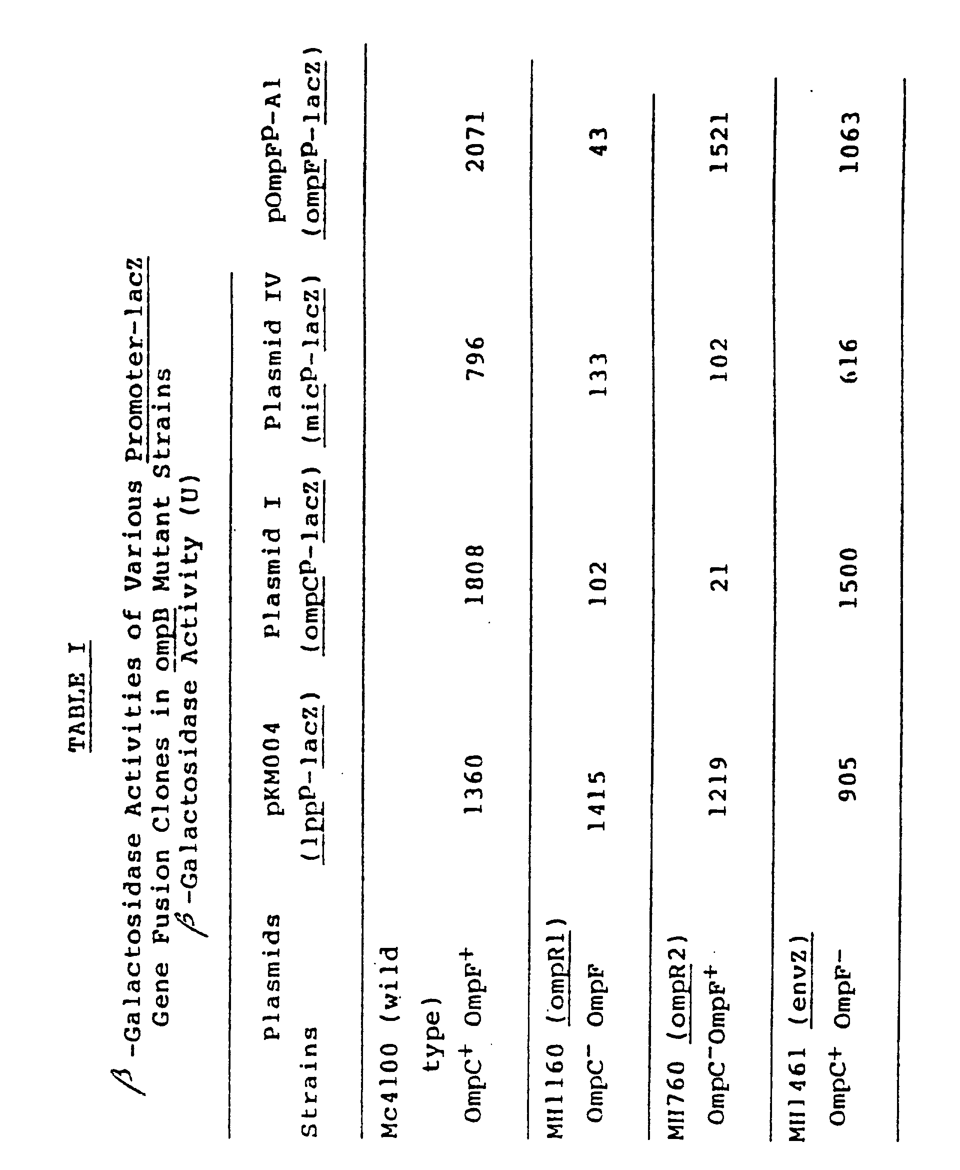

Various ompB mutant strains, MC4100 (F⁻lacVI69 araDI39 rspL thiA tibB relA; wild type), MHII60 [ompBI0I (amRI) mutant from MC4100] MH760 [ampB427 (ompR2) mutant from MC4100], MH1461 [tpoll - (envZ) mutant from MC4100] were transformed by various promoter-lacZ gene fusion clones. Cells were grown in 10 ml of nutrient broth at 37°C to Klett unit of 1.2. 100 µl of the cultures were used for β-galactosidase activity measurement according to the method of Miller, H.J., in Experiments of Molecular Genetics (ed. Miller, H.J.) 352-355 (Cold Spring Harbor Laboratory, New York (1972)). Plasmid pK004 was derived from pKM005 and pKM004 contains the Ipp (the gene for outer membrane lipoprotein) promoter fused to the lacZ gene. Plasmid I and IV are described in Fig. 1c. Plasmid pOmpFP-Al contains the lacZ gene under the control of the ompF promoter.

As shown in Table I, the lacZ gene under micF control (plasmid IV in Fig. 1 C) produces *β*-galactosidase in the same manner as the lacZ gene under ompC promoter control (plasmid I in Fig. 1C): high *β*-galactosidase activity was found in both the wild type and envZ⁻ strains but low activity was observed in ompR1⁻ and ompR2⁻ mutants. On the other hand, the lacZ gene under the control of the ompF promoter was not expressed in the ompR1⁻ cells. In addition, lacZ gene under the control of the lipoprotein promoter, used as a control, was expressed in all strains. These results indicate that the micF gene is regulated by the ompB locus in the same fashion as the ompC gene. It is interesting to note that the lacZ gene under the control of the ompF promoter is constitutively expressed in the envZ⁻ (Ompc⁺ OmpF⁻) strain. This suggests that the OmpF- phenotype of this envZ⁻ strain is due to the inhibition of translation of the ompF mRNA by micRNA.

### Promoters of the micF and ompC Genes

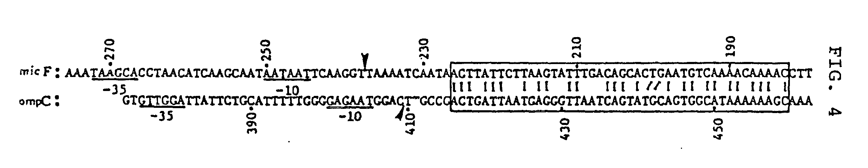

Since both the micF and ompC genes appear to be regulated by the ompB locus, the promoters of these genes should have sequence homologies. In order to search for the homologies, the transcription initiation site for the ompC gene was first determined by S1-nuclease mapping. Major transcription initiation takes place at the T residues at position 410 and 411 (Fig. 2; also see Fig. 4).

In Fig. 4 there is shown the homologous sequences between the micF and the ompC genes. Nucleotide numbers correspond to those in Fig. 2. The sequences in the box show the homologous sequences between the two genes. Bars between the two sequences indicate the identical bases. The arrows indicate the transcription initiation sites. The -10 and -35 regions are underlined.

The -10 regions for the micF and ompC genes are assigned as AATAAT (nucleotides 250 to 245 in Fig. 2) and GAGAAT (nucleotides 400 to 405 in Fig. 2), respectively (Fig. 4), both of which show good homology to the consensus sequence, TATAAT. RNA polymerase recognition sites, (-35 regions), for the micF and ompC genes are also assigned as TAAGCA and TTGGAT, respectively (Fig. 4), both of which show 50% homology to the consensus sequence, TTGACA. However, no significant sequence homologies are found between the micF promoter of 63 bp (nucleotides 300 to 238) and the ompC promoter (nucleotides 301 to 400 in Fig. 2). On the other hand, homologous seqences are found in the 5'-end regions of both the transcripts as shown in Fig. 4. Twenty-eight out of 44 bases are homologous (64% homology), and these regions are probably the sites recognized by OmpR protein. It is interesting to note that a homologous sequence to these sequences has also been found in the 5'-end untranslated region of ompF mRNA. Binding experiments of the micF gene and the ompC gene with purified OmpR protein are now in progress.

As indicated hereinabove, regulation of gene expression in E. coli is generally controlled at the level of transcription. It has been well established that expression of some genes are suppressed by their specific repressors or activated by their specific inducers. Positive protein factors such as cAMP receptor protein and OmpR protein are also known to regulate gene expression at the level of transcription. Another transcriptional regulatory mechanism is attenuation which plays an important role in controlling expression of operations involved in the biosynthesis of various amino acids of other compounds, see Kolter, R. & Yanofsky, C. Ann. Rev. Genet. 16, 113-134 (1982).

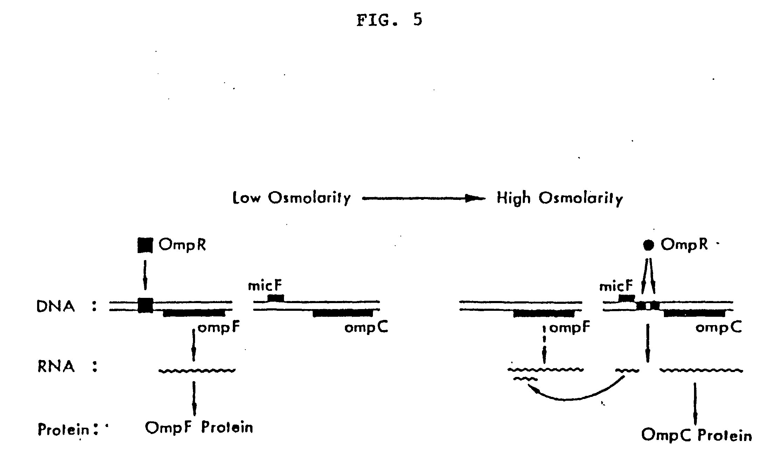

In addition, some proteins have been shown to regulate gene expression at the level of translation. The results herein demonstrate the regulation of bacterial gene expression at the level of translation by means of a complementary RNA factor to the translational start region. This novel regulatory mechanism mediated by micRNA is illustrated in Fig. 5.

Fig. 5 illustrates a possible model for the role of micF RNA. OmpR protein binds to the ompF gene under the low osmolarity and promotes the production of OmpF protein. Under the high osmolarity, OmpR protein binds to both the micF and the ompC genes. The micF RNA thus produced hybridizes with the ompF mmRNA to arrest its translation.

The possibility that micRNA blocks the expression of the ompF gene at the level of transcription has not been ruled out. However, this is highly unlikely since the lacZ gene fused with the ompF promoter was expressed in the envZ⁻ cells (OmpC⁺ OmpF⁻; Table 1). In this case lacZ expression is probably due to the inability of lacZ mRNA transcribed from the clone to form a stable hybrid with micRNA. Furthermore, if micRNA is able to bind the nonsense strand of the ompF gene, it would more likely stimulate gene expression rather than block it, since the binding would make the ompF gene more accessible to RNA polymerase.

Regulation by micRNA appears to be an extremely efficient way to block production of a specific protein without hampering other protein production. At present, the relative ratio between micRNA and ompC production is not known (*β*-galactosidase activities in Table I do not necessarily reflect their accurate promoter activities, since the promoter regions were not inserted in the same fashion, see Fig. 1c). However, it is reasonable to assume that the micRNA and the ompC are produced coordinately. Therefore, when OmpC protein is produced, micRNA is produced in the same manner. micRNA then blocks the production of OmpF protein proportionally, so that the total amount of OmpC plus OmpF protein is constant.

The binding of micRNA to the ribosome-binding site and the initiation codon is a very effective way to block the translation of the particular mRNA. A similar mechanism has been proposed to explain a translational block in a mutant of bacteriophage T7. It was suggested that the sequence of the 3'-end of a mutant mRNA hybridizes with its own ribosome-binding site to block translation, see Saito, H. & Richardson, C.C. Cell 27, 533-542 (1981). It seems reasonable that the micRNA regulatory system may be a general regulatory phenomenon in E. coli and in other organisms including eukaryotes. It is a particularly attractive mechanism to very rapidly stop the formation of a protein or to control the ratio of one protein with another. RNA species may have additional roles in the regulation of various celluar activities. In fact, small RNA species have been shown to be involved in the regulation of DNA replication of some plasmids.

In view of the accompanying disclosure it is seen that there is presented in accordance with the practices of this invention a powerful tool and technique for regulating gene expression. Gene expression in accordance with the practices of this invention is regulated by incorporating in or associating with the genetic material of an organism or cellular material (which may possess only its native genetic material or which may have been genetically altered by the deletion of native genetic material or the addition of foreign genetic material) DNA, which upon transcription along with the genetic material of said organism or cellular material, produces an oligoribonucleotide or polyribonucleotide RNA complementary to and/or capable of hybridizing with an mRNA produced by the genetic material of the organism or cellular material so that expression or translation of said RNA is inhibited or prevented.

The regulation of the gene expression of an organism or cellular material in accordance with the practices of this invention is carried out in a transformed organism or cellular material wherein along with the genetic material of said organism or cellular material there is incorporated therein or associated therewith DNA, which upon transcription along with the genetic material of said organism or cellular material, produces an oligoribonucleotide or polyribonucleotide RNA complementary to and/or capable of binding or hybridizing with an mRNA produced by the genetic material of said organism or cellular material so that the expression or translation of said mRNA is inhibited or prevented.

In the practices of this invention the DNA material or molecule which, upon transcription in a transformed organism or cellular material containing said DNA material or molecule, produces an oligoribonucleotide or polyribonucleotide RNA complementary to and/or capable of binding or hybridizing with a mRNA produced by the genetic material of said organism or cellular material, may be incorporated or associated with the genetic material of the organism to be transformed by transforming the organism or cellular material with the DNA material or molecule per se directly or by incorporating the DNA material in a plasmid or virus or viral vector and then transforming the organism or cellular material with the plasmid and/or viral vector. The DNA material or molecule may be inserted directly into the nucleus containing the genetic material of the organism or cellular material. The DNA material or molecule effecting transformation of the organism or cellular material may be inserted into the organism through the membrane thereof into the cytoplasm or fluid content of the organism or cellular material into association with the genetic or chromosomal DNA material characterizing the organism. Where desired, convenient, or practical, microinjection may be employed to insert the DNA material or molecule into the organism or cellular material to be transformed, such as into the nucleus or cytoplasm of the organism. It is usually convenient to incorporate or associate the DNA material or molecule with the genetic material of the organism or cellular material to be transformed by transfer of the DNA material or molecule through the membrane encompassing the organism or cellular material.

### Construction of an Artificial Mic Gene

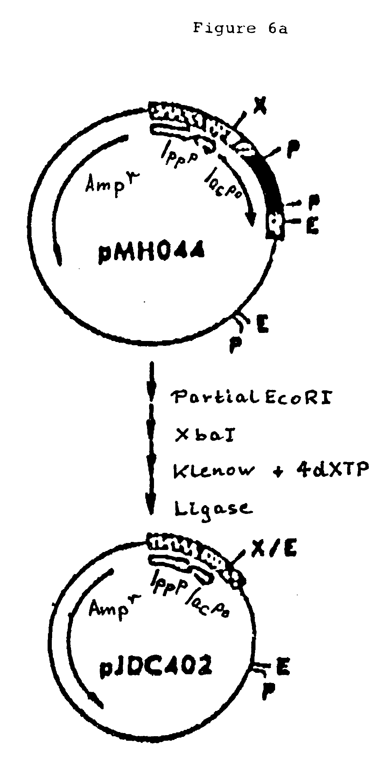

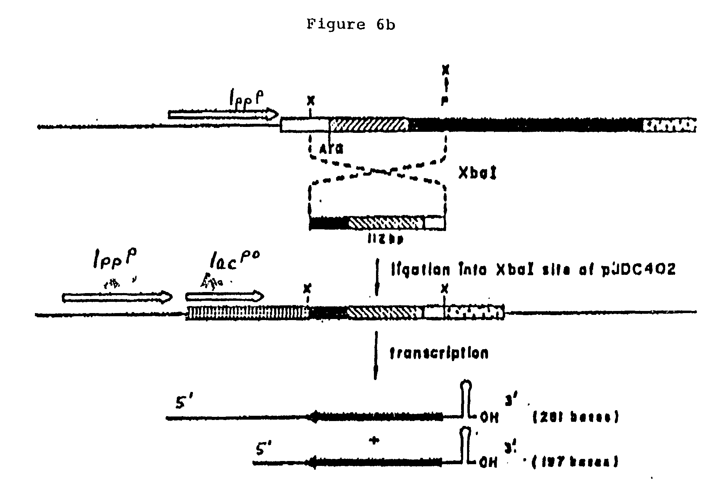

The micF gene produces a 174-base RNA that blocks production of the OmpF protein. This small RNA has two stem-and-loop structures, one at the 3'-end and the other at the 5'-end. Since these structures are considered to play an important role for the function of the micRNA, it was attempted to use these features in the construction of an artificial mic system using the gene for the major outer membrane lipoprotein (Ipp) cloned in an inducible expression vector, pIN-II, see Nakamura et al, "Construction of Versatile Expression Cloning Vehicles Using the Lipoprotein Gene of Escherichia coli", EMBO J. 1, 771-775 (1982). pIN-II vectors are high expression vectors that have the lac^{po} downstream of the lipoprotein promoter, thus allowing high level inducible expression of an inserted gene. The pIN-II promoter was fused to the Ipp gene at a unique Xbal site immediately upstream of the Shine-Dalgarno sequence of the Ipp mRNA. The resulting plasmid was designated as pYM140. When the expression of the Ipp gene, in pYM140, is induced by isopropyl-*β*-D-thiogalactoside (IPTG), a lac inducer, the RNA transcript derived from the Ipp gene has a possible stem-and-loop sructure (at the 5' end). Immediately upstream of the unique Xbal site, see Fig. 6-A, is another stable stem-and-loop structure at its 3' end. The latter loop is derived from the p-independent transcription termination signal of the Ipp gene. The construction of a general mic cloning vector, pJDC402 was achieved by removing the DNA fragement in pMHO44 between the two loops as shown in Fig. 6 -A. An Rsal site immediately upstream of the termination site was changed to an EcoRI site by partial digestion of pYM140 followed by insertion of an EcoRI linker. The resulting plasmid, pMHO44 was partially digested with EcoRI, followed by a complete digestion with Xbal. The single stranded portions of the linear DNA fragment were filled in with DNA polymerase I (large fragment), and then treated with T4 DNA ligase, resulting in the formation of the plasmid, pJDC402, which lost the fragment between the Xbal and the Rsal sites. As a result of this procedure, both an EcoRI and an Xbal site were recreated at the junction. Thus the unique Xbal site can serve as the insertion site for any DNA fragment, and the RNA transcript from the artificial mic gene produces an RNA which has a similar structure to the micF RNA; the portion derived from the inserted DNA is sandwiched by two loop structures, one at the 5' and one at the 3'-end.

The following is a more detailed description of Fig. 6 -A and Fig. 6-B. As illustrated in Fig. 6-A for the construction of pJDC402, restriction sites are indicated as follows: X, Xbal; P, Pvull; E, EcoRI. Ipp^{P} and lac^{po} are the lipoprotein promoter and the lactose promoter operator, respectively. Amp^{r} is the Ampicillin resistance gene. Cross hatches represent the lipoprotein promoter. Solid dots represent the lactose promoter operator. Slashes indicate the lipoprotein signal sequence, and the solid bar represents the coding region for the mature portion of the lipoprotein. The open dots represent the transcription termination region derived from the Ipp gene. The open bar represents the 5' nontranslated region of the lipoprotein mRNA.

In Fig. 6-B for the construction of mic (lpp) pJDC412, open arrows represent promoters. The Pvull site was converted to an Xbal site by inserting an Xbal linker (TCTAGAG). This fragment was inserted into the unique Xbal site of pJDC402 in the reverse orientation forming pJDC412. a and b show the mic(lpp) RNAs initiating at the lpp and lac promoters, respectively.

### Construction of the mic(lpp) Gene

Using this mic cloning vector, pJDC402, it was first attempted to create a mic system for the Ipp gene of E. coli, in order to block the synthesis of the lipoprotein upon induction of the mic(lpp) gene. For this purpose it is necessary to first isolate the DNA fragment containing the Shine-Dalgarno sequence for ribosome binding, and the coding region for the first few amino acid residues of prolipoprotein. To do this the Pvull site immediately after the coding region of prolipoprotein signal peptide was changed to an Xbal site by inserting an Xbal linker at this position. The resulting plasmid was then digested with Xbal, and the 112-bp XbaI-XbaI (originally PvuII-XbaI) fragment was purified. This fragment encompassing the Shine-Dalgarno sequence and the coding region for the first 29 amino residues from the amino terminus of prolipoprotein was purified. This fragment was then inserted into the unique Xbal site of pJDC402 in the opposite orientation from the normal Ipp gene. The resulting plasmid, designated as pJDC412, is able to produce mic(lpp) RNA, an RNA transcript complementary to the Ipp mRNA, upon induction with IPTG.

It should be pointed out that another important feature of the mic expression vector, pJDC402, is that it contains a Hinfl site immediately upstream of the Ipp promoter and another one immediately downstream of the transcription termination site. These two Hinfl sites can be used to remove a DNA fragment containing the entire mic transcription unit which can then be inserted back into the unique Pvull site of the vector. In this manner, the entire mic gene can be duplicated in a single plasmid. One would expect a plasmid containing two identical mic genes to produce twice as much micRNA as a plasmid containing a single mic gene. Such a plasmid was constructed containing two mic(Ipp) genes and designated as pJDC422.

### Expression of the mic(Ipp) Gene

In order to examine the effect of the artificial mic(Ipp) RNA, cells were pulse-labeled for one minute, with [³⁵S]-methionine,one hour after induction of the mic(Ipp) RNA with 2mM IPTG. The cells harboring the vector, pJDC402, produce the same amount of lipoprotein either in the absence or the presence of the induce, IPTG, as quantitated by densitometric scanning of the autoradiogram and normalizing. Lipoprotein production was reduced approximately two-fold in the case of cells carrying pJDC412 in the absence of IPTG and approximately 16-fold in the presence or IPTG. The reduction in lipoprotein synthesis in the absence of IPTG is considered to be due to incomplete repression of the mic(lpp) gene. In the case of cells carrying pJDC422, where the mic(lpp) gene was duplicated, lipoprotein production is now reduced 4-fold in the absence of IPTG, and 31-fold in the presence of IPTG. These results clearly demonstrate that the production of the artificial mic(Ipp) RNA inhibits lipoprotein production, and that the inhibition is proportional to the amount of the mic(lpp) RNA produced. It should be noted that the mic(lpp) RNA is specifically blocking the production of lipoprotein, and that it does not block the production of any other proteins except for OmpC protein. The fact that the induction of the mic(lpp) gene reduces the production of the OmpC plus OmpF proteins was found to be due to unusual homology between the lpp and the ompC gene as discussed hereinafter.

There are several mechanisms by which the mic inhibition may occur. One mechanism is that the micRNA binds to the mRNA preventing the ribosome from binding the mRNA. Other possible mechanisms include: destabilization of the mRNA, attenuation of the mRNA due to premature termination of transcription, or inhibition of transcription initiation. If the inhibitory effect of the micRNA is solely at the level of attenuation or transcription initiation one would expect the mic effect to be somewhat delayed due to the fact that the functional half-life of the lipoprotein mRNA is 12 minutes. Therefore, it was examined how rapidly lipoprotein production is inhibited upon induction of the mic(lpp) RNA by pulse-labeling E. coli JA221/F'lacI^{q} harboring pJDC412, with [³⁵S]-methionine at various time points after induction with IPTG. It was determined that lipoprotein production was maximally inhibited by 16-fold within 5 minutes after the addition of IPTG. This result indicates that inhibition of lipoprotein production is primarily due to the binding of the mic(Ipp) RNA to the Ipp mRNA, resulting in the inhibition of translation of the Ipp mRNA and/or destabilization of the mRNA.

### Ipp mRNA Production in the Presence of mic(Ipp) RNA

It appeared interesting to examine whether the mic(Ipp) RNA also affects the level of the Ipp mRNA, since the expression of the micF gene substantially reduced the amount of the ompF mRNA. For this purpose, there was isolated total cellular RNA one hour after the induction of the mic(Ipp) gene with IPTG. The RNA preparation was analyzed after electrophoresis in a formaldehyde agarose gel and subsequently transferred onto nitrocellulose paper. The paper was then hybridized with a probe specific to the mic(lpp) RNA, or to the Ipp mRNA. There was also used a probe specific for the ompA mRNA as an internal control. Again pJDC402 shows no difference in the production of the Ipp mRNA in the absence or presence of IPTG. Due to the fact that the double stranded primer used to make the probe for these experiments contains a portion of the lac operon, the probes hybridize to any transcript containing the lac promoter such as the mic(lpp) RNA from JDC412 and the short nonsense transcript from pJDC402. Cells harboring pJDC412 contain a reduced amount of the Ipp mRNA in the absence of IPTG and a greatly reduced amount of the Ipp mRNA in the presence of IPTG. There was shown the production of the mic(lpp) RNA in the absence and the presence of IPTG in cells harboring pJDC412. Therefore, even in the absence of IPTG, a significant amount of the mic(Ipp) RNA is produced, which is consistent with the results of the lipoprotein production observed earlier. The fact that the Ipp mRNA disappears upon induction of the mic(Ipp) RNA indicates that the mechanism of action of the micRNA is not solely at the level of translation. Tests demonstrated there are two mic(Ipp) RNAs of different sizes. The sizes of these transcripts were estimated to be 281 and 197 bases, which correspond to transcripts initiating at the lipoprotein promoter (the larger RNA) and initiating at the lac promoter (the smaller RNA).

### Inhibition of OmpC Production with the mic(ompC) Gene

It was also possible to achieve an almost complete inhibition of OmpC synthesis by artificially constructing mic(ompC) genes. The first construct, pAM320, carrying two mic(ompC) genes gives rise to an RNA molecule complementary to 20 nucleotides of the leader region and 100 nucleotides of the coding region of the ompC mRNA. This was achieved by changing the unique Bglll site in the ompC structural gene and the Mnll site, 20 nucleotides upstream of the ATG initiation codon to Xbal sites. The resulting 128-bp Xbal fragment was then inserted into pJDC402 in the opposite orientation from the OmpC gene and a second copy of the mic(ompC) gene was introduced in a manner similar to that described for the pJDC422 construction. The resulting plasmid, pAM320, has the second mic(ompC) gene inserted in the orientation opposite to the first one. Reversing the orientation of the second mic gene did not change the expression or stability of the plasmid. A second construct, pAM321, was designed to extend the complementarity between the micRNA and the ompC mRNA to include a longer leader sequence than in the case of pAM320, 72 nucleotides of the leader region instead of 20. This plasmid was constructed as described for pAM320, except that the Mnll site changed to an Xbal site was located 72 nucleotides bp upstream of the ompC initiation codon.

Commassie Brilliant Blue stained gel patterns of the outer membrane proteins isolated from E. coli JA221/F'lacl^{q} harboring the mic cloning vector pJDC402, pAM320 and pAM321 were obtained. The effect of the addition of IPTG was clearly seen by the appearance of *β*-galactosidase. The induction of the mic(ompC) RNA from pAM320 caused a substantial decease (approximately 5-fold) in OmpC production, compared to pJDC402. Induction of the longer mic(ompC) RNA from pAM321 decreased OmpC synthesis more dramatically (approximately 20-fold compared to pJDC402). OmpC production could hardly be detected in the cells harboring pAM321 when they were pulse-labeled for one minute after a one-hour induction with IPTG. In the same experiment, OmpC synthesis decreased approximately 7-fold when the mic(ompC) gene in cells harboring pAM320 was induced with IPTG. Marked decreases in OmpC expression were also observed when plasmids containing single copies of the mic(ompC) genes here induced. Again, the longer mic(ompC) gene had a greater effect. The increased efficiency of mic-mediated inhibition with pAM321 may indicate that the effectiveness of the micRNA function is related to the extent of complementarity to the 5'-end of the mRNA.

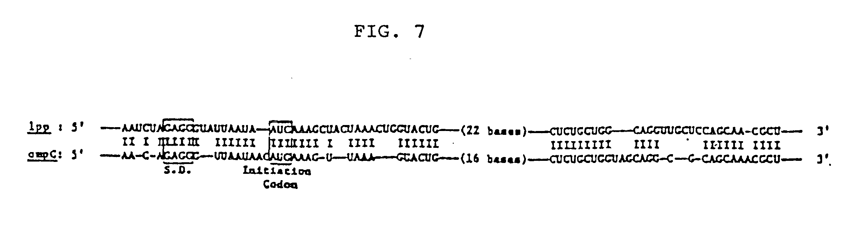

It was interesting to note that the synthesis of either of the mic(ompC) RNAs described above caused a decrease not only in OmpC synthesis but also in lipoprotein synthesis. This inhibitory effect of the mic(ompC) RNA on lipoprotein production appears to be due to the unexpected homology between the Ipp mRNA sequence and the ompC mRNA as illustrated in Fig. 7. This feature explains why pAM320 and pAM321 are exerting a mic effect on lipoprotein production. Such an explanation would predict that induction of the mic(lpp) RNA from pJDC412 and pJDC422 should decrease the synthesis of the OmpC protein, and this was found to be the case.

In Fig. 7, there is illustrated a region of homology between the lpp mRNA (top line) and the ompC mRNA (bottom line). Bars connect identical bases. Both mic(ompC) RNAs have the potential to hybridize across this homologous region. The Shine-Dalgarno Sequences (S.D.) and AUG initiation codons are boxed.

### Inhibition of OmpA Production with mic(ompA) RNA

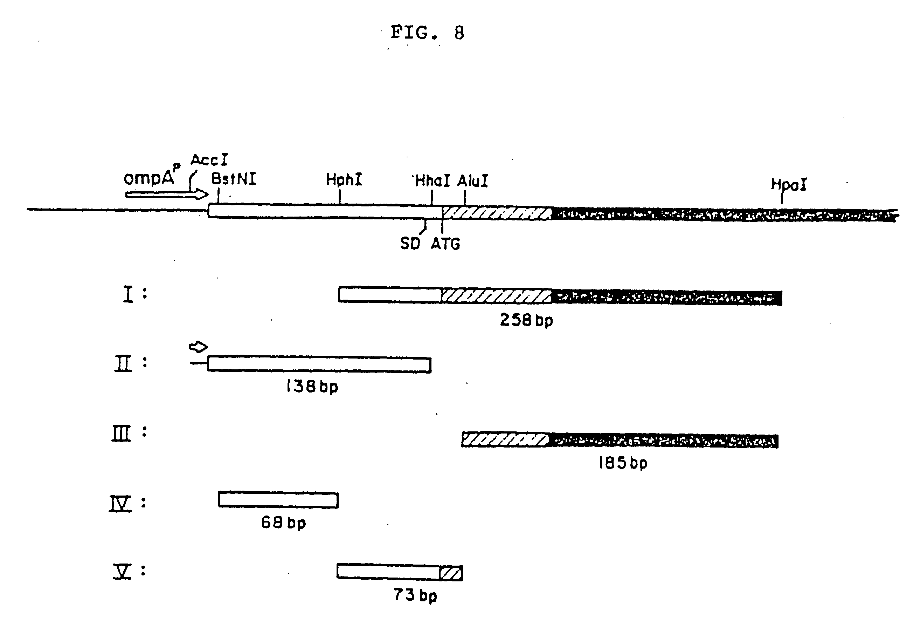

In an effort to determine what components contribute to the effectiveness of a micRNA, several mic genes were constructed from the ompA gene. The ompA gene was selected for this because the leader and the coding regions of the ompA mRNA have been characterized extensively. Five DNA fragments (see I through V of Fig. 8 were individually cloned into the Xbal site of pJDC402 in the orientation promoting the production of mic(ompA) RNAs. The resulting mic(ompA) plasmids containing fragments I-V were designated as pAM301, pAM307, pAM313, pAM314, and pAM318, respectively. Each plasmid contains only one copy of the described mic(ompA) gene.

In Fig. 8, the top line shows the structure of the E. coli ompA gene. The arrow represents the promoter and the open bar represents the region encoding the 5'-leader region of the ompA mRNA. The slashed bar and shaded bar represent the portions of the ompA gene encoding the signal sequence and the mature OmpA protein, respectively. Restriction fragment I (Hphl-Hpal) was inserted into the Xbal site of pJDC402, see Fig. 6-A, in the orientation opposite from that depicted here, as outlined in Fig. 6-B for mic(lpp), to create the plasmid, pAM301. The other mic(ompA) plasmids were similarly constructed from: fragment II, pAM307; fragment III, pAM313; fragment IV, pAM314; fragment V, pAM318. The positions of the Shine-Dalgarno sequence (SD), ATG initiation codon (ATG), and relevant restriction sites are shown.

E. coli JA221/F'lacI^{q} containing each of the mic(ompA) plasmids was pulse-labeled with [³⁵S]-methionine for one minute with and without a one-hour prior preincubation with IPTG. Electrophorectic patterns of the outer membrane proteins isolated from these cultures were obtained. The autoradiographs revealed that each of the five mic(ompA) genes is capable of inhibiting OmpA synthesis. The mic(ompA) genes appear to be less effective than the mic(lpp) and mic(ompC) genes described earlier, but this problem was circumvented by increasing the mic(ompA) gene dosage.

The plasmid pAM301, encoding an mRNA complementary to a 258 base region of the ompA mRNA encompassing the translation initiation site (fragment I in Fig.8) was found to inhibit OmpA synthesis by approximately 45 percent. A similar inhibition was obtained with pAM307, by approximately 51 percent. This plasmid contains fragment II (see Fig. 8 ) which does not include any DNA sequences corresponding to the ompA structural gene. The inhibition by pAM307 was not surprising because the mic(ompC) experiments described earlier showed that increased complementarity to the 5'-leader region of the mRNA was more effective in micRNA-mediated inhibition. On the other hand, pAM313, which produces a micRNA that is complementary only to the portion of the ompA structural gene covered by fragment III (See Fig. 8 which spans the coding region for amino acid residues 4 through 45 of pro-OmpA, was also effectively able to inhibit OmpA synthesis by approximately 54 percent, indicating that the micRNA does not need to hybridize to the initiation site for protein synthesis and/or to the 5'-leader region of the target mRNA in order to function. This was also confirmed using mic(lpp) genes. Two mic(lpp) RNAs which were complementary to only the coding region of the lpp mRNA have also been found to inhibit lipoprotein production. The effect of the mic(Ipp) genes in pJDC413 and pJDC414 which were constructed from the lpp structural gene fragments coding for amino acid residues 3 to 29, and 43 to 63 of prolipoprotein, respectively, were observed. Both pJDC413 and pJDC414, however, exhibit only a 2-fold inhibition of lipoprotein synthesis indicating that a DNA fragment covering the translation initiation site (which caused a 16-fold inhibition) is more effective in the case of the mic(Ipp) genes.

Fragment IV (see Fig. 8) was chosen to test the effectiveness of a micRNA complementary only to the 5' leader region of the ompA mRNA. The resulting construct pAM314, synthesizes a micRNA complementary to a 68-base stretch of the ompA mRNA leader located 60 bases upstream of the AUG initiation codon. pAM314 exhibits a very weak mic effect, inhibiting OmpA synthesis by only about 18 percent. The significant differences in the mic effect between fragments II and IV (see Fig. 8) clearly demonstrates that the complementary interaction within the region of the mRNA that interacts with the ribosome i.e., the Shine-Dalgarno sequence and/or the coding region, is very important for the effective mic function, although it is not absolutely required. It is also interesting to note that shortening the mic(ompA) gene from fragment I to V had little effect on its efficiency, a 45 percent compared to a 48 percent decrease, respectively.

In order to construct a plasmid capable of inhibiting OmpA synthesis more effectively than those discussed above, plasmids were constructed containing more than one mic(ompA) gene. The plasmid, pAM307 and its derivatives, pAM319 and pAM315, were compared. The latter two plasmids contain two and three copies of the mic(ompA) gene in pAM307, respectively. While pAM307 inhibited OmpA synthesis by approximately 41 percent, pAM315 and pAM319 inhibited OmpA synthesis by 69 percent and 73 percent, respectively.

The results presented hereinabove clearly demonstrate that the artificial mic system and techniques of this invention can be used for specifically regulating the expression of a gene of interest. In particular, the inducible mic system for a specific gene is a novel and very effective way to study the function of a gene. If the gene is essential, conditional lethality may be achieved upon the induction of the mic system; somewhat similar to a temperature-sensitive mutation. It should be noted, however, that the mic system blocks the synthesis of the specific protein itself while temperature-sensitive mutations block only the function of the protein without blocking its synthesis.

From this invention, the following has become evident:
(a) The production of an RNA transcript (micRNA) that is complementary to a specific mRNA inhibits the expression of that mRNA.
(b) The production of a micRNA specifically blocks the expression of only those genes which share complementarity to the micRNA.
(c) The induction of micRNA production blocks the expression of the specific gene very rapidly in less than the half-life of the mRNA.
(d) The micRNA also reduces the amount of the specific mRNA in the cell, as was found when the natural micF gene was expressed, as well as when the artificially constructed mic(lpp) gene was expressed in the present invention.
(e) There is a clear effect of gene dosage; the more a micRNA is produced, the more effectively the expression of the target gene is blocked.

In the practices of this invention, it appears that micRNAs complementarity to regions of the mRNA known to interact with ribosomes are the most effective. Using the Ipp gene as an example, it appears that a mic(lpp) RNA that can hybridize to the Shine-Dalgarno sequence and the translation-initiation site of the Ipp mRNA inhibits lipoprotein synthesis more efficiently than one which cannot. However, for the ompA gene, micRNAs complementary to both the Shine-Dalgarno sequence and the translation-initiation site, just the Shine-Dalgarno sequence or the structural gene alone were equally effective.

For some genes, such as ompC and Ipp, the region of the gene encompassing the translation-initiation site may not contain a unique sequence, and micRNA induction results in the inhibition of the production of more than one protein. In these cases, another region of the gene may be used to construct the mic gene. The length of the micRNA is another variable to be considered. The longer mic(ompC) RNA was 4-fold more effective at inhibiting OmpC production than the shorter mic(ompC) RNA. This indicates that higher specificity may be achieved by using longer micRNAs. It should be noted that the inhibition of lipoprotein expression by the mic(ompC) RNA was less effective with the longer mic(ompC) RNA, in spite of the fact that the region of the two mic(ompC) RNAs complementary to the lipoprotein mRNA is the same. In contrast to the mic(ompC) genes, length did not appear to be a significant factor for the mic(ompA) RNA-mediated inhibition of OmpA production. In addition, the secondary structure of the micRNA most likely plays an important role in micRNA function.

There are several mechanisms by which the micRNA may function to inhibit expression of the specific gene. It is most likely that the micRNA primarily acts by binding to the mRNA, thereby preventing the interaction with ribosomes as proposed earlier. This hypothesis is supported by the fact that the mic(lpp) RNA inhibited lipoprotein production much faster than the time expected if only transcription was affected based on the half-life of the Ipp mRNA. Concerning how micRNA causes a reduction in the amount of lipoprotein mRNA, a plausible model to explain this reduction is that the mRNA is less stable when ribosomes are not traversing the entire mRNA. Another possible model to explain this reduction in mRNA level is that complementary hybrid formation between the micRNA and the mRNA causes premature termination of transcription or destabilization of the mRNA. Alternatively, the micRNA may directly inhibit the initiation of transcription, or cause pausing of mRNA elongation in a manner similar to that described for the function of a small complementary RNA species in ColEI replication, see Tomizawa et al., The importance of RNA secondary structure in ColEI primer formation. Cell 31, 575-583 (1982).

The mic system of this invention has great potential in its application, in prokaryotic as well as eukaryotic cells, to block, permanently or upon induction, the expression of various toxic or harmful genes such as drug resistance genes, oncogenes, and phage or virus genes and the expression of other genes.

In the development and demonstration of the practices of this invention as described herein, the following materials and procedures were employed.

### Strain and Medium

E. coli JA221 (hsdr leuB6 lacY thi recA ΔtrpE5)F' (lacl^{q} proAB lacZYA) was used in all experiments. This strain was grown in M9 medium (J.H. Miller, Experiments in Molecular Genetics. Cold spring Harbor Laboratory, Cold Spring Harbor, New York (1972)) supplemented with 0.4 percent glucose, 2 *µ* g/ml thiamine, 40 *µ*g/ml each of leucine and tryptophan, and 50 *µ*g/ml ampicillin, unless otherwise indicated.

### Materials

Restriction enzymes were purchased from either Bethesda Research Laboratories or New England BioLabs. T4 DNA ligase and E. coli DNA polymerase I (large fragment) were purchased from Bethesda Research Laboratories. All enzymes were used in accordance with the instructions provided by the manufacturer. Xbal linkers (CTCTAGAG) were purchased from New England BioLabs.

### DNA Manipulation

Plasmids pJDC402, pJDC412, and pJDC422 were constructed as described herein and in Fig. 6. Plasmids pJDC413 and pJDC414 were constructed by isolating the 80-bp Alul fragment from the Ipp gene encoding amino acid residues 3 through 29 of prolipoprotein for pJDC413 and the 58-bp Alul fragment encoding amino acid residues 43 through 63 or prolipoprotein for pJDC414. The fragments were blunt end ligated into pJDC402 which was first digested with Xbal followed by treatment with DNA polymerase I (large fragment).

The isolation of the appropriate ompC fragments for mic(ompC) construction involved a subcloning step due to the absence of suitable unique restriction sites between the ompC promoter and structural gene. Two derivatives of the ompC containing plasmid, pMY150, lacking either the 471-bp XbaI-MnII ompC promoter containing fragment (pDR001 and pDR002, respectively), but containing an Xbal site in its place, were isolated. The unique BgIII sites in each of these plasmids were changed to Xbal sites by treatment with DNA polymerase I (large fragment) and ligation with synthetic Xbal linkers. Following Xbal digestion, the 123-bp Xbal fragment from pDR001 and the 175-bp Xbal fragment from pDR002 were individually isolated and cloned into the Xbal site of pJDC402 to create pAM308 and pAM309, respectively. pAM320 contains the Einfl fragment covering the mic(ompC) gene isolated from pAM308 cloned into the Pvull site of pAM308. pAM321 was similarly, constructed from pAM309 to also contain two mic(ompC) genes.

The mic(ompA) plasmids pAM301, pAM307, pAM313, pAM314, and pAM318 were constructed as described in a manner similar to the construction of the mic(lpp) and the mic(ompC) genes. To construct pAM319, the HinfI fragment containing the mic(ompA) gene was isolated from pAM307 and inserted back into the Pvull site of pAM307. pAM315 was constructed in the same manner as pAM319 except that it contains two Hinfl fragments inserted into the Pvull site of pAM307.

### Analysis of outer membrane protein production

E. coli JA221/F'lacI^{q} carrying the appropriate plasmid were grown to a Klett-Summerson colorimeter reading of 30, at which time IPTG was added to a final concentration of 2 mM. After one additional hour of growth (approximately one doubling), 50 µCi of [³⁵S]-Methionine (Amersham, 1000 Ci/mMole) was added to 1 ml of the culture. The mixture was then incubated with shaking for one minute, at which time the labeling was terminatd by addition of 1 ml ice cold stop solution (20 mM sodium phosphate, [pH 7.1] containing 1 percent formaldehyde, and 1 mg/ml methionine). Cells were washed once with 10 mM sodium phosphate, pH 7.1, suspended in 1 ml of the same buffer, and sonicated with a Heat Systems Ultrasonics sonicator model W-220E with a cup horn adapter for 3 minutes (in 30 second pulse). Unbroken cells were removed by low speed centrifugation prior to collecting the outer membrane. Cytoplasmic membranes were solubilized during a 30 minute incubation at room temperature in the presence Of 0.5 percent sodium lauroyl sarcosinate and the outer membrane fraction was precipitated by centrifugation at 105,000 X g for 2 hours.

Lipoprotein and OmpA were analyzed by Tris-SDS polyacrylamide gel electrophoresis (SDS-PAGE). To analyze OmpC production, urea-SDS polyacrylamide gel electrophoreis (urea-SDS-PAGE) was used. Proteins were dissolved in the sample buffer and the solution was incubated in a boiling water bath for 8 minutes prior to gel application. The autoradiographs of dried gels were directly scanned by a Shimadzu densitometer. To determine relative amounts of the band of interest, the ratio of the area of the peak of interest to the area of an unaffected protein peak, was determined for each sample.

### RNA Analysis

Cells were grown and labeled with [³H]-uridine, then cell growth was stopped by rapidly chilling the culture on ice for loss than 5 minutes. The cells were collected by centrifugation at 8000 rpm for 5 minutes. RNA was isolated using the following procedure. The cells were quickly resuspended in hot lysis solution (10 mM Tris-HCI [pH 8.0], 1 mM EDTA, 350 mM NaCl, 2 percent SDS and 7 M urea) with vigorous vortexing for 1 minute. The mixture was immediately extracted, twice with phenol: chloroform (1:1) and twice with chloroform alone. One tenth volume of 3 M sodium acetate (pH 5.2) was added to the mixture and 3 volumes of ethanol was added to precipitate the RNA. The precipitate was then dissolved in TE buffer (10 mM Tris-HCI [pH 7.5], 1 mM EDTA). For gel electrophoresis, equal counts were loaded in each lane. The RNA was separated on a 1.5 percent agarose gel containing 6 percent formaldehyde. The running buffer was 20 mM MOPS (3-[N-morpholino]propanesulfonic acid [Sigma]), 5 mM sodium acetate and 1 mM EDTA, pH 7.0.

RNA was transferred to nitrocellulose paper. M13 hybridization probes specific for the mic(Ipp) RNA and Ipp mRNA were individually constructed by cloning the 112-bp Xbal fragment shown in Fig 1-B into M13 mp9 in the appropriate orientation. A probe specific for the ompA mRNA was constructed by inserting a 1245-bp XbaI-EcoRI fragment (originally an EcoRV-PSTI fragment) into M13 mp10 and the probes were labeled.

## Claims (Claims for the following Contracting State(s): BE, CH, DE, FR, GB, IT, LI, LU, NL, SE)

1. An artificial nucleic acid construct which, upon introduction into a cell containing a gene, antagonizses the function of said gene, said artificial nucleic acid construct containing the following nucleic acid segments:
(a) a transcriptional promoter segment;
(b) a transcription termination segment; and therebetween
(c) a nucleic acid sequence segment;
whereby transcription of the nucleic acid sequence segment produces a ribonucleotide sequence which does not naturally occur in the cell, is complementary to at least a portion of a ribonucleotide sequence transcribed by said gene, and said non-naturally occurring ribonucleotide sequence antagonizes the function of said gene.

2. A nucleic acid construct of claim 1 wherein said nucleic acid sequence segment encodes a ribonucleotide sequence complementary to a 5' end non-coding portion of said ribonucleotide acid sequence transcribed by said gene.

3. A nucleic acid construct of claim 1 wherein said nucleic acid sequence segment encodes a ribonucleotide sequence complementary to a ribosome binding portion of said ribonucleotide acid sequence transcribed by said gene.

4. A nucleic acid construct of claim 1 wherein said nucleic acid sequence segment encodes a ribonucleotide sequence complementary to the translation initiation portion of said ribonucleotide sequence transcribed by said gene.

5. An artificial nucleic acid construct which, upon introduction into a cell containing a gene, antagonizes the function of said gene, said artificial nucleic acid construct containing the following nucleic acid segments:
(a) a transcriptional promoter segment;
(b) a transcription termination segment; and
(c) a segment of said gene, said gene segment located between said promoter segment and said termination segment and being inverted with respect to said promoter segment and said termination segment, the polarity of said inverted gene segment being the same as that of said promoter segment and said termination segment, whereby transcription of the inverted gene segment occurs in a direction opposite to the direction of transcription of the gene to thereby antagonize the function of said gene.

6. A nucleic acid construct of any one of claims 1 to 5 wherein said gene is an oncogene.

7. A nucleic acid construct of any one of claims 1 to 5 wherein said gene is a viral gene.

8. A nucleic acid construct of any one of claims 1 to 7 wherein said gene encodes a protein.

9. A nucleic acid construct of any one of claims 1 to 8 wherein said transcriptional promoter segment comprises an inducible promoter.

10. A nucleic acid construct of any one of claims 5 to 9 wherein said gene segment includes the 5' non-coding region of said gene.

11. A nucleic acid construct of any one of claims 5 to 9 wherein said gene segment includes the ribosome binding portion of said gene.

12. A nucleic acid construct of any one of claims 5 to 9 wherein said gene segment includes the translation initiation portion of said gene.

13. A vector having incorporated therein a nucleic acid construct according to any one of claims 1 to 12.

14. A vector according to claim 13 wherein said vector is a plasmid.

15. A vector according to claim 13 wherein said vector is a viral vector.

16. A nucleic acid construct of any one of claims 1 to 12 or a vector according to any one of claims 13-15 which is incorporated in, or associated with, the chromosomal genetic material of an organism or cellular material.

17. A nucleic acid construct or a vector according to claim 16 incorporated in, or associated with, the chromosomal genetic material in the nucleus of said organism or cellular material.

18. A pharmaceutical composition which comprises the nucleic acid construct of any one of claims 1 to 12 or the vector according to any one of claims 13 to 15.

19. A method of antagonizing the function of a gene in a microorganism comprising:
(a) constructing an artificial nucleic acid construct according to any one of claims 1 to 12 or a vector according to any one of claims 13 to 15 which, upon transcription in said microorganism, produces RNA transcript complementary to RNA transcript produced by said gene; and
(b) introducing said artificial nucleic acid construct or vector into the microorganism containing said gene.

20. A method of antagonizing the function of a gene in a cell comprising:
(a) constructing an artificial nucleic acid construct according to any one of claims 1 to 12 or a vector according to any one of claims 13 to 15 which, upon transcription in said cell, produces RNA transcript complementary to RNA transcript produced by said gene; and
(b) introducing said artificial nucleic acid construct or vector into the cell containing said gene;
wherein said method does not include a method for treatment of the human or animal body by therapy or a diagnostic method practised on the human or animal body.

21. The method of claims 19 to 20 wherein said RNA transcript transcribed by said artificial nucleic acid construct or vector does not naturally occur in said cell.

22. A micro-organism containing a nucleic acid construct according to any one of claims 1 to 12 or a vector according to any one of claims 13 to 15.

23. The micro-organism according to claim 22, which is a bacterium or a virus.

## Claims (Claims for the following Contracting State(s): AT)

1. Process for the preparation of an artificial nucleic acid construct which, upon introduction into a cell containing a gene, antagonizes the function of said gene, which comprises combining the following nucleic acid segments:
(a) a transcriptional promoter segment;
(b) a transcription termination segment; and therebetween
(c) a nucleic acid sequence segment;
whereby transcription of the nucleic acid sequence segment produces a ribonucleotide sequence which does not naturally occur in the cell, is complementary to at least a portion of a ribonucleotide sequence transcribed by said gene, and said non-naturally occurring ribonucleotide sequence antagonizes the function of said gene.

2. Process according to claim 1 whereby said nucleic acid sequence segment of the nucleic acid construct encodes a ribonucleotide sequence complementary to a 5' end noncoding portion of said ribonucleotide acid sequence transcribed by said gene.

3. Process according to claim 1 whereby said nucleic acid sequence segment of the nucleic acid construct encodes a ribonucleotide sequence complementary to a ribosome binding portion of said ribonucleotide acid sequence transcribed by said gene.

4. Process according to claim 1 whereby said nucleic acid sequence segment of the nucleic acid construct encodes a ribonucleotide sequence complementary to the translation initiation portion of said ribonucleotide sequence transcribed by said gene.

5. Process for the preparation of an artificial nucleic acid construct which, upon introduction into a cell containing a gene, antagonizes the function of said gene, which comprises combining the following nucleic acid segments:
(a) a transcriptional promoter segment;
(b) a transcription termination segment; and
(c) a segment of said gene, said gene segment being located between said promoter segment and said termination segment and being inverted with respect to said promoter segment and said termination segment, the polarity of said inverted gene segment being the same as that of said promoter segment and said termination segment, whereby transcription of the inverted gene segment occurs in a direction opposite to the direction of transcription of the gene to thereby antagonize the function of said gene.

6. A process according to any one of claims 1 to 5 wherein said gene is an oncogene.

7. A process according to any one of claims 1 to 5 wherein said gene is a viral gene.

8. A process according to any one of claims 1 to 7 wherein said gene encodes a protein.

9. A process according to any one of claims 1 to 8 wherein said transcriptional promoter segment comprises an inducible promoter.

10. A process according to any one of claims 5 to 9 wherein said gene segment includes the 5' non-coding region of said gene.

11. A process according to any one of claims 5 to 9 wherein said gene segment includes the ribosome binding portion of said gene.

12. A process according to any one of claims 5 to 9 wherein said gene segment includes the translation initiation portion of said gene.

13. A vector having incorporated therein a nucleic acid construct obtained according to any one of claims 1 to 12.

14. A vector according to claim 13 wherein said vector is a plasmid.

15. A vector according to claim 13 wherein said vector is a viral vector.

16. A process according to any one of claims 1 to 12 or a vector according to any one of claims 13 to 15 wherein the nucleic acid construct obtained is to be incorporated in, or associated with, the chromosomal genetic material of an organism or cellular material.

17. A process according to claim 16 wherein the nucleic acid construct or vector obtained is to be incorporated in, or associated with, the chromosomal genetic material in the nucleus of said organism or cellular material.

18. A process for the preparation of a pharmaceutical composition which comprises combining the nucleic acid construct of any one of claims 1 to 12 or the vector according to any one of claims 13 to 15 with a pharmaceutically acceptable carrier.

19. A method of antagonizing the function of a gene in a microorganism comprising:
(a) constructing an artificial nucleic acid construct according to any one of claims 1 to 12 or a vector according to any one of claims 13 to 15 which, upon transcription in said microorganism, produces RNA transcript complementary to RNA transcript produced by said gene; and
(b) introducing said artificial nucleic acid construct or vector into the microorganism containing said gene.

20. A method of antagonizing the function of a gene in a cell comprising:
(a) constructing an artificial nucleic acid construct according to any one of claims 1 to 12 or a vector according to any one of claims 13 to 15 which, upon transcription in said cell, produces RNA transcript complementary to RNA transcript produced by said gene; and
(b) introducing said artificial nucleic acid construct or vector into the cell containing said gene;
wherein said method does not include a method for treatment of the human or animal body by therapy or a diagnostic method practised on the human or animal body.

21. The method of claims 19 to 20 wherein said RNA transcript transcribed by said artificial nucleic acid construct or vector does not naturally occur in said cell.

22. A microorganism containing a nucleic acid construct according to any one of claims 1 to 12 or a vector according to any one of claims 13 to 15.

23. The microorganism according to claim 22, which is a bacterium or a virus.

## Patentansprüche (Patentansprüche für folgende(n) Vertragsstaat(en): BE, CH, DE, FR, GB, IT, LI, LU, NL, SE)

1. Künstliches Nucleinsäurekonstrukt, das nach dem Einführen in eine Zelle, die ein Gen enthält, gegen die Funktion dieses Gens wirkt, wobei das künstliche Nucleinsäurekonstrukt nachstehende Nucleinsäureabschnitte enthält:
(a) einen Promotorabschnitt für die Transkription;
(b) einen Terminationsabschnitt für die Transkription; und dazwischen
(c) einen Nucleinsäuresequenz-Abschnitt,
wobei die Transkription des Nucleinsäuresequenz-Abschnitts eine Ribonucleotidsequenz erzeugt, die in der Zelle nicht natürlich vorkommt, mindestens zu einem Teil einer von dem Gen transkribierten Ribonucleotidsequenz komplementär ist, und wobei die nicht-natürlich vorkommende Ribonucleotidsequenz gegen die Funktion des Gens wirkt.

2. Nucleinsäurekonstrukt nach Anspruch 1, wobei der Nucleinsäuresequenz-Abschnitt eine Ribonucleotidsequenz codiert, die zu einem nicht-codierenden Teil am 5'-Ende der von dem Gen transkribierten Ribonucleotidsequenz komplementär ist.

3. Nucleinsäurekonstrukt nach Anspruch 1, wobei der Nucleinsäuresequenz-Abschnitt eine Ribonucleotidsequenz codiert, die zu einem Ribosom-bindenden Teil der durch das Gen transkribierten Ribonucleotidsequenz komplementär ist.

4. Nucleinsäurekonstrukt nach Anspruch 1, wobei der Nucleinsäuresequenz-Abschnitt eine Ribonucleotidsequenz codiert, die zu dem Translationsinitiations-Teil der von dem Gen transkribierten Ribonucleotidsequenz komplementär ist.

5. Künstliches Nucleinsäurekonstrukt, das nach Einführen in eine Zelle, die ein Gen enthält, gegen die Funktion des Gens wirkt, wobei das künstliche Nucleinsäurekonstrukt die nachstehenden Nucleinsäureabschnitte enthält:
(a) einen Promotorabschnitt für die Transkription;
(b) einen Terminationsabschnitt für die Transkription; und
(c) einen Abschnitt des Gens, wobei sich der Genabschnitt zwischen dem Promotorabschnitt und dem Terminationsabschnitt befindet und in Bezug auf den Promotorabschnitt und Terminationsabschnitt umgekehrt ist, wobei die Polarität des umgekehrten Genabschnitts die gleiche ist wie die des Promotorabschnitts und des Terminationsabschnitts, und wobei die Transkription des umgekehrten Genabschnitts in eine Richtung entgegen der Richtung der Transkription des Gens auftritt, um dadurch gegen die Funktion des Gens zu wirken.

6. Nucleinsäurekonstrukt nach einem der Ansprüche 1 bis 5, wobei das Gen ein Oncogen ist.

7. Nucleinsäurekonstrukt nach einem der Ansprüche 1 bis 5, wobei das Gen ein virales Gen ist.

8. Nucleinsäurekonstrukt nach einem der Ansprüche 1 bis 7, wobei das Gen ein Protein codiert.

9. Nucleinsäurekonstrukt nach einem der Ansprüche 1 bis 8, wobei der Promotorabschnitt für die Transkription einen induzierbaren Promotor umfaßt.

10. Nucleinsäurekonstrukt nach einem der Ansprüche 5 bis 9, wobei der Genabschnitt den 5'-nicht-codierenden Bereich des Gens einschließt.

11. Nucleinsäurekonstrukt nach einem der Ansprüche 5 bis 9, wobei der Genabschnitt den Ribosom-bindenden Teil des Gens einschließt.

12. Nucleinsäurekonstrukt nach einem der Ansprüche 5 bis 9, wobei der Genabschnitt den Translationsinitiations-Teil des Gens einschließt.

13. Vektor, der ein eingebautes Nucleinsäurekonstrukt nach einem der Ansprüche 1 bis 12 aufweist.

14. Vektor nach Anspruch 13, wobei der Vektor ein Plasmid ist.

15. Vektor nach Anspruch 13, wobei der Vektor ein viraler Vektor ist.

16. Nucleinsäurekonstrukt nach einem der Ansprüche 1 bis 12 oder Vektor nach einem der Ansprüche 13 bis 15, eingebaut in oder verbunden mit dem chromosomalen genetischen Material eines Organismus oder zellulären Materials.

17. Nucleinsäurekonstrukt oder Vektor nach Anspruch 16, eingebaut in oder verbunden mit dem chromosomalen genetischen Material im Kern des Organismus oder zellulären Materials.

18. Arzneimittel, umfassend das Nucleinsäurekonstrukt nach einem der Ansprüche 1 bis 12 oder den Vektor nach einem der Ansprüche 13 bis 15.

19. Verfahren zum Gegenwirken der Funktion eines Gens in einem Mikroorganismus, umfassend:
(a) die Konstruktion eines künstlichen Nucleinsäurekonstrukts nach einem der Ansprüche 1 bis 12 oder eines Vektors nach einem der Ansprüche 13 bis 15, das bzw. der nach der Transkription in dem Mikroorganismus ein RNA-Transkript erzeugt, das zu dem von dem Gen erzeugten RNA-Transkript komplementär ist; und
(b) das Einführen des künstlichen Nucleinsäurekonstrukts oder Vektors in den Mikroorganismus, der das Gen enthält.

20. Verfahren zum Gegenwirken der Funktion eines Gens in einer Zelle, umfassend:
(a) die Konstruktion eines künstlichen Nucleinsäurekonstrukts nach einem der Ansprüche 1 bis 12 oder eines Vektors nach einem der Ansprüche 13 bis 15, das bzw. der nach der Transkription in der Zelle ein RNA-Transkript erzeugt, das zu dem von dem Gen erzeugten RNA-Transkript komplementär ist; und
(b) das Einführen des künstlichen Nucleinsäurekonstrukts oder Vektors in die Zelle, die das Gen enthält;
wobei das Verfahren kein Verfahren zur therapeutischen Behandlung eines Menschen oder Tieres oder kein am Menschen oder Tier durchzuführendes diagnostisches Verfahren einschließt.

21. Verfahren nach Anspruch 19 oder 20, wobei das von dem künstlichen Nucleinsäurekonstrukt oder Vektor transkribierte RNA-Transkript in der Zelle nicht natürlich vorkommt.

22. Mikroorganismus, enthaltend ein Nucleinsäurekonstrukt nach einem der Ansprüche 1 bis 12 oder einen Vektor nach einem der Ansprüche 13 bis 15.

23. Mikroorganismus nach Anspruch 22, der ein Bacterium oder ein Virus ist.

## Patentansprüche (Patentansprüche für folgende(n) Vertragsstaat(en): AT)

1. Verfahren zur Herstellung eines künstlichen Nucleinsäurekonstrukts, das nach Einführen in eine Zelle, die ein Gen enthält, gegen die Funktion des Gens wirkt, umfassend das Zusammenfügen der nachstehenden Nucleinsäureabschnitte:
(a) einen Promotorabschnitt für die Transkription;
(b) einen Terminationsabschnitt für die Transkription; und dazwischen
(c) einen Nucleinsäuresequenz-Abschnitt,
wobei die Transkription des Nucleinsäuresequenz-Abschnitts eine Ribonucleotidsequenz erzeugt, die in der Zelle nicht natürlich vorkommt, mindestens zu einem Teil einer von dem Gen transkribierten Ribonucleotidsequenz komplementär ist, und wobei die nicht-natürlich vorkommende Ribonucleotidsequenz gegen die Funktion des Gens wirkt.

2. Verfahren nach Anspruch 1, wobei der Nucleinsäuresequenz-Abschnitt des Nucleinsäurekonstrukts eine Ribonucleotidsequenz codiert, die zu einem nicht-codierenden Teil am 5'-Ende der von dem Gen transkribierten Ribonucleotidsequenz komplementär ist.

3. Verfahren nach Anspruch 1, wobei der Nucleinsäuresequenz-Abschnitt des Nucleinsäurekonstrukts eine Ribonucleotidsequenz codiert, die zu einem Ribosom-bindenden Teil der von dem Gen transkribierten Ribonucleotidsequenz komplementär ist.

4. Verfahren nach Anspruch 1, wobei der Nucleinsäuresequenz-Abschnitt des Nucleinsäurekonstrukts eine Ribonucleotidsequenz codiert, die zu dem Translationsinitiations-Teil der von dem Gen transkribierten Ribonucleotidsequenz komplementär ist.

5. Verfahren zur Herstellung eines künstlichen Nucleinsäurekonstrukts, das nach Einführen in eine Zelle, die ein Gen enthält, gegen die Funktion des Gens wirkt, umfassend das Zusammenfügen der nachstehenden Nucleinsäureabschnitte:
(a) einen Promotorabschnitt für die Transkription;
(b) einen Terminationsabschnitt für die Transkription; und
(c) einen Abschnitt des Gens, wobei sich der Genabschnitt zwischen dem Promotorabschnitt und dem Terminationsabschnitt befindet und in Bezug auf den Promotorabschnitt und Terminationsabschnitt umgekehrt ist, wobei die Polarität des umgekehrten Genabschnitts die gleiche ist wie die des Promotorabschnitts und des Terminationsabschnitts, und wobei die Transkription des umgekehrten Genabschnitts in eine Richtung entgegen der Richtung der Transkription des Gens auftritt, um dadurch gegen die Funktion des Gens zu wirken.

6. Verfahren nach einem der Ansprüche 1 bis 5, wobei das Gen ein Oncogen ist.

7. Verfahren nach einem der Ansprüche 1 bis 5, wobei das Gen ein virales Gen ist.

8. Verfahren nach einem der Ansprüche 1 bis 7, wobei das Gen ein Protein codiert.

9. Verfahren nach einem der Ansprüche 1 bis 8, wobei der Promotorabschnitt für die Transkription einen induzierbaren Promotor umfaßt.

10. Verfahren nach einem der Ansprüche 5 bis 9, wobei der Genabschnitt den 5'-nicht-codierenden Bereich des Gens einschließt.

11. Verfahren nach einem der Ansprüche 5 bis 9, wobei der Genabschnitt den Ribosom-bindenden Teil des Gens einschließt.

12. Verfahren nach einem der Ansprüche 5 bis 9, wobei der Genabschnitt den Translationsinitiations-Teil des Gens einschließt.

13. Vektor, der ein nach einem der Ansprüche 1 bis 12 erhaltenes, eingebautes Nucleinsäurekonstrukt aufweist.

14. Vektor nach Anspruch 13, wobei der Vektor ein Plasmid ist.

15. Vektor nach Anspruch 13, wobei der Vektor ein viraler Vektor ist.

16. Verfahren nach einem der Ansprüche 1 bis 12 oder Vektor nach einem der Ansprüche 13 bis 15, wobei das erhaltene Nucleinsäurekonstrukt in das chromosomale genetische Material eines Organismus oder zellulären Materials einzubauen ist oder damit zu verbinden ist.

17. Verfahren nach Anspruch 16, wobei das erhaltene Nucleinsäurekonstrukt oder der Vektor in das chromosomale genetische Material im Kern des Organismus oder zellulären Materials einzubauen ist oder damit zu verbinden ist.

18. Verfahren zur Herstellung eines Arzneimittels, umfassend das Vereinigen des Nucleinsäurekonstrukts nach einem der Ansprüche 1 bis 12 oder des Vektors nach einem der Ansprüche 13 bis 15 mit einem pharmazeutisch verträglichen Träger.

19. Verfahren zum Gegenwirken der Funktion eines Gens in einem Mikroorganismus, umfassend:
(a) die Konstruktion eines künstlichen Nucleinsäurekonstrukts nach einem der Ansprüche 1 bis 12 oder eines Vektors nach einem der Ansprüche 13 bis 15, das bzw. der nach der Transkription in dem Mikroorganismus ein RNA-Transkript erzeugt, das zu dem von dem Gen erzeugten RNA-Transkript komplementär ist; und
(b) das Einführen des künstlichen Nucleinsäurekonstrukts oder Vektors in den Mikroorganismus, der das Gen enthält.

20. Verfahren zum Gegenwirken der Funktion eines Gens in einer Zelle, umfassend:
(a) die Konstruktion eines künstlichen Nucleinsäurekonstrukts nach einem der Ansprüche 1 bis 12 oder eines Vektors nach einem der Ansprüche 13 bis 15, das bzw. der nach der Transkription in der Zelle ein RNA-Transkript erzeugt, das zu dem von dem Gen erzeugten RNA-Transkript komplementär ist; und
(b) das Einführen des künstlichen Nucleinsäurekonstrukts oder Vektors in die Zelle, die das Gen enthält;
wobei das Verfahren kein Verfahren zur therapeutischen Behandlung eines Menschen oder Tieres oder kein am Menschen oder Tier durchzuführendes diagnostisches Verfahren einschließt.

21. Verfahren nach Anspruch 19 oder 20, wobei das von dem künstlichen Nucleinsäurekonstrukt oder Vektor transkribierte RNA-Transkript in der Zelle nicht natürlich vorkommt.

22. Mikroorganismus, enthaltend ein Nucleinsäurekonstrukt nach einem der Ansprüche 1 bis 12 oder einen Vektor nach einem der Ansprüche 13 bis 15.

23. Mikroorganismus nach Anspruch 22, der ein Bacterium oder ein Virus ist.

## Revendications (Revendications pour l'(les) Etat(s) contractant(s) suivant(s): BE, CH, DE, FR, GB, IT, LI, LU, NL, SE)

1. Construction d'acide nucléique artificielle qui, lors de l'introduction dans une cellule contenant un gène, antagonise la fonction de ce gène, cette construction d'acide nucléique artificielle contenant les segments d'acide nucléique suivants :
(a) un segment promoteur transcriptionnel;
(b) un segment de terminaison de transcription; et entre ceux-ci
(c) un segment de séquence d'acide nucléique;
de sorte que la transcription du segment de séquence d'acide nucléique produit une séquence ribonucléotidique qui n'apparaît pas naturellement dans la cellule, est complémentaire d'au moins une partie d'une séquence ribonucléotidique transcrite par le gène précité, et la séquence ribonucléotidique n'apparaissant pas naturellement antagonise la fonction dudit gène.

2. Construction d'acide nucléique suivant la revendication 1, **caractérisée en ce que** le segment de séquence d'acide nucléique code pour une séquence ribonucléotidique complémentaire d'une partie non codante d'extrémité 5' de la séquence d'acide ribonucléotidique transcrite par le gène précité.

3. Construction d'acide nucléique suivant la revendication 1, **caractérisée en ce que** le segment de séquence d'acide nucléique code pour une séquence ribonucléotidique complémentaire d'une partie de liaison de ribosome de la séquence d'acide ribonucléotidique transcrite par le gène précité.

4. Construction d'acide nucléique suivant la revendication 1, **caractérisée en ce que** le segment de séquence d'acide nucléique code pour une séquence ribonucléotidique complémentaire de la partie d'initiation de la traduction de la séquence ribonucléotidique transcrite par le gène précité.

5. Construction d'acide nucléique artificielle qui, lors de l'introduction dans une cellule contenant un gène, antagonise la fonction de ce gène, cette construction d'acide nucléique artificielle contenant les segments d'acide nucléique suivants :
(a) un segment promoteur transcriptionnel;
(b) un segment de terminaison de transcription; et
(c) un segment du gène précité, ce segment de gène étant localisé entre le segment promoteur et le segment de terminaison et étant inversé par rapport à ce segment promoteur et ce segment de terminaison, la polarité du segment de gène inversé étant la même que celle du segment promoteur et du segment de terminaison, de sorte que la transcription du segment de gène inversé se produit dans une direction opposée à la direction de transcription du gène pour antagoniser ainsi la fonction dudit gène.

6. Construction d'acide nucléique suivant l'une quelconque des revendications 1 à 5, **caractérisée en ce que** le gène est un oncogène.

7. Construction d'acide nucléique suivant l'une quelconque des revendications 1 à 5, **caractérisée en ce que** le gène est un gène viral.

8. Construction d'acide nucléique suivant l'une quelconque des revendications 1 à 7, **caractérisée en ce que** le gène code pour une protéine.

9. Construction d'acide nucléique suivant l'une quelconque des revendications 1 à 8, **caractérisée en ce que** le segment promoteur transcriptionnel comprend un promoteur inductible.

10. Construction d'acide nucléique suivant l'une quelconque des revendications 5 à 9, **caractérisée en ce que** le segment de gène comprend la région non codante 5' dudit gène.

11. Construction d'acide nucléique suivant l'une quelconque des revendications 5 à 9, **caractérisée en ce que** le segment de gène comprend la partie de liaison de ribosome dudit gène.

12. Construction d'acide nucléique suivant l'une quelconque des revendications 5 à 9, **caractérisée en ce que** le segment de gène comprend la partie d'initiation de la traduction dudit gène.

13. Vecteur incorporant une construction d'acide nucléique suivant l'une quelconque des revendication 1 à 12.

14. Vecteur suivant la revendication 13, **caractérisé en ce que** ledit vecteur est un plasmide.

15. Vecteur suivant la revendication 13, **caractérisé en ce que** ledit vecteur est un vecteur viral.

16. Construction d'acide nucléique suivant l'une quelconque des revendications 1 à 12 ou vecteur suivant l'une quelconque des revendications 13 à 15, qui est incorporé dans ou associé à la matière génétique chromosomique d'un organisme ou d'une matière cellulaire.

17. Construction d'acide nucléique ou vecteur suivant la revendication 16, qui est incorporé dans ou associé à la matière génétique chromosomique dans le noyau de cet organisme ou de cette matière cellulaire.

18. Composition pharmaceutique qui comprend la construction d'acide nucléique suivant l'une quelconque des revendications 1 à 12 ou le vecteur suivant l'une quelconque des revendications 13 à 15.

19. Procédé d'antagonisation de la fonction d'un gène dans un micro-organisme comprenant :
(a) la construction d'une construction d'acide nucléique artificielle suivant l'une quelconque des revendications 1 à 12 ou d'un vecteur suivant l'une quelconque des revendications 13 à 15, qui, lors de la transcription dans le micro-organisme, produit un transcript d'ARN complémentaire du transcript d'ARN produit par le gène; et
(b) l'introduction de la construction d'acide nucléique artificielle ou du vecteur dans le micro-organisme contenant ledit gène.

20. Procédé d'antagonisation de la fonction d'un gène dans une cellule comprenant :
(a) la construction d'une construction d'acide nucléique artificielle suivant l'une quelconque des revendications 1 à 12 ou d'un vecteur suivant l'une quelconque des revendications 13 à 15, qui, lors de la transcription dans la cellule, produit un transcript d'ARN complémentaire du transcript d'ARN produit par le gène; et
(b) l'introduction de la construction d'acide nucléique artificielle ou du vecteur dans la cellule contenant ledit gène;
**caractérisé en ce que** ce procédé ne comprend pas de méthode de traitement du corps humain ou animal par thérapie ou une méthode de diagnostic pratiquée sur le corps humain ou animal.

21. Procédé suivant l'une ou l'autre des revendications 19 et 20, **caractérisé en ce que** le transcript d'ARN transcrit par la construction d'acide nucléique artificielle ou le vecteur précité n'apparaît pas naturellement dans ladite cellule.

22. Micro-organisme contenant une construction d'acide nucléique suivant l'une quelconque des revendications 1 à 12 ou un vecteur suivant l'une quelconque des revendications 13 à 15.

23. Micro-organisme suivant la revendication 22, qui est une bactérie ou un virus.

## Revendications (Revendications pour l'(les) Etat(s) contractant(s) suivant(s): AT)

1. Procédé pour la préparation d'une construction d'acide nucléique artificielle qui, lors de l'introduction dans une cellule contenant un gène, antagonise la fonction de ce gène, qui comprend la combinaison des segments d'acide nucléique suivants :
(a) un segment promoteur transcriptionnel;
(b) un segment de terminaison de transcription; et entre ceux-ci
(c) un segment de séquence d'acide nucléique;
de sorte que la transcription du segment de séquence d'acide nucléique produit une séquence ribonucléotidique qui n'apparaît pas naturellement dans la cellule, est complémentaire d'au moins une partie d'une séquence ribonucléotidique transcrite par le gène précité, et la séquence ribonucléotidique n'apparaissant pas naturellement antagonise la fonction dudit gène.

2. Procédé suivant la revendication 1, grâce auquel le segment de séquence d'acide nucléique de la construction d'acide nucléique code pour une séquence ribonucléotidique complémentaire d'une partie non codante d'extrémité 5' de la séquence d'acide ribonucléotidique transcrite par le gène précité.

3. Procédé suivant la revendication 1, grâce auquel le segment de séquence d'acide nucléique de la construction d'acide nucléique code pour une séquence ribonucléotidique complémentaire d'une partie de liaison de ribosome de la séquence d'acide ribonucléotidique transcrite par le gène précité.

4. Procédé suivant la revendication 1, grâce auquel le segment de séquence d'acide nucléique de la construction d'acide nucléique code pour une séquence ribonucléotidique complémentaire de la partie d'initiation de la traduction de la séquence ribonucléotidique transcrite par le gène précité.

5. Procédé pour la préparation d'une construction d'acide nucléique artificielle qui, lors de l'introduction dans une cellule contenant un gène, antagonise la fonction de ce gène, qui comprend la combinaison des segments d'acide nucléique suivants :
(a) un segment promoteur transcriptionnel;
(b) un segment de terminaison de transcription; et
(c) un segment du gène précité, ce segment de gène étant localisé entre le segment promoteur et le segment de terminaison et étant inversé par rapport à ce segment promoteur et ce segment de terminaison, la polarité du segment de gène inversé étant la même que celle du segment promoteur et du segment de terminaison, de sorte que la transcription du segment de gène inversé se produit dans une direction opposée à la direction de transcription du gène pour antagoniser ainsi la fonction dudit gène.

6. Procédé suivant l'une quelconque des revendications 1 à 5, **caractérisé en ce que** le gène est un oncogène.

7. Procédé suivant l'une quelconque des revendications 1 à 5, **caractérisé en ce que** le gène est un gène viral.

8. Procédé suivant l'une quelconque des revendications 1 à 7, **caractérisé en ce que** le gène code pour une protéine.

9. Procédé suivant l'une quelconque des revendications 1 à 8, **caractérisé en ce que** le segment promoteur transcriptionnel comprend un promoteur inductible.

10. Procédé suivant l'une quelconque des revendications 5 à 9, **caractérisé en ce que** le segment de gène comprend la région non codante 5' dudit gène.

11. Procédé suivant l'une quelconque des revendications 5 à 9, **caractérisé en ce que** le segment de gène comprend la partie de liaison de ribosome dudit gène.

12. Procédé suivant l'une quelconque des revendications 5 à 9, **caractérisé en ce que** le segment de gène comprend la partie d'initiation de la traduction dudit gène.

13. Vecteur incorporant une construction d'acide nucléique obtenue suivant l'une quelconque des revendications 1 à 12.

14. Vecteur suivant la revendication 13, **caractérisé en ce que** ledit vecteur est un plasmide.

15. Vecteur suivant la revendication 13, **caractérisé en ce que** ledit vecteur est un vecteur viral.

16. Procédé suivant l'une quelconque des revendications 1 à 12 ou vecteur suivant l'une quelconque des revendications 13 à 15, **caractérisé en ce que** la construction d'acide nucléique obtenue est à incorporer dans ou à associer à la matière génétique chromosomique d'un organisme ou d'une matière cellulaire.

17. Procédé suivant la revendication 16, **caractérisé en ce que** la construction d'acide nucléique ou le vecteur obtenu est à incorporer dans ou à associer à la matière génétique chromosomique dans le noyau de cet organisme ou de cette matière cellulaire.

18. Procédé pour la préparation d'une composition pharmaceutique, qui comprend la combinaison de la construction d'acide nucléique suivant l'une quelconque des revendications 1 à 12 ou du vecteur suivant l'une quelconque des revendications 13 à 15 avec un support pharmaceutiquement acceptable.

19. Procédé d'antagonisation de la fonction d'un gène dans un micro-organisme comprenant :
(a) la construction d'une construction d'acide nucléique artificielle suivant l'une quelconque des revendications 1 à 12 ou d'un vecteur suivant l'une quelconque des revendications 13 à 15, qui, lors de la transcription dans le micro-organisme, produit un transcript d'ARN complémentaire du transcript d'ARN produit par le gène; et
(b) l'introduction de la construction d'acide nucléique artificielle ou du vecteur dans le micro-organisme contenant ledit gène.

20. Procédé d'antagonisation de la fonction d'un gène dans une cellule comprenant :
(a) la construction d'une construction d'acide nucléique artificielle suivant l'une quelconque des revendications 1 à 12 ou d'un vecteur suivant l'une quelconque des revendications 13 à 15, qui, lors de la transcription dans la cellule, produit un transcript d'ARN complémentaire du transcript d'ARN produit par le gène; et
(b) l'introduction de la construction d'acide nucléique artificielle ou du vecteur dans la cellule contenant ledit gène;
**caractérisé en ce que** ce procédé ne comprend pas de méthode de traitement du corps humain ou animal par thérapie ou une méthode de diagnostic pratiquée sur le corps humain ou animal.

21. Procédé suivant l'une ou l'autre des revendications 19 et 20, **caractérisé en ce que** le transcript d'ARN transcrit par la construction d'acide nucléique artificielle ou le vecteur précité n'apparaît pas naturellement dans ladite cellule.

22. Micro-organisme contenant une construction d'acide nucléique suivant l'une quelconque des revendications 1 à 12 ou un vecteur suivant l'une quelconque des revendications 13 à 15.

23. Micro-organisme suivant la revendication 22, qui est une bactérie ou un virus.
